# EUROPEAN PATENT APPLICATION

(11) **EP 1 916 526 A1**
(43) Date of publication of application: **30.04.2008**
(21) Application number: 06123005.8
(22) Date of filing: 26.10.2006
(51) Int. Cl.: G01N 33/60

(54) **Method for diagnostic and therapeutic target discovery by combining isotopic and isobaric labels**

(71) Applicant: Koninklijke Philips Electronics N.V., 5621 BA Eindhoven (NL)
(72) Inventor: The designation of the inventor has not yet been filed
(74) Representative: van Wermeskerken, Stephanie Christine

(57) **Abstract**

The present invention describes isotopic and isobaric labelling reagents suitable for double labelling different individual protein samples. After double labelling of the individual protein samples, the labelled polypeptides in an individual sample have a unique combination of an isotopic and an isobaric label. Thereafter all samples are pooled. Peptides from the pooled samples are isolated and analysed by mass spectrometry for determining the relative concentration of each differentially labelled polypeptide

## Description

### FIELD OF THE INVENTION

The present invention provides methods and tools for the screening of targets in by mass spectrometric approaches. More particularly the methods and tools of the invention allow the parallel screening of multiple samples using liquid chromatography mass spectrometry using a combination of isotopic and isobaric peptide labelling procedures.

### BACKGROUND OF THE INVENTION

The analysis and identification of proteins from highly complex mixtures demands tremendous resolving power. Two methods commonly used to resolve such mixtures are 2-Dimensional Gel Electrophoresis (2D-GE) and (2-Dimensional) Liquid Chromatography ((2D)-LC).

High-resolution 2D-GE was introduced in by Klose (1975) Humangenetik 26, 231-243 and O'Farrell (1975), J. Biol. Chem. 250, 4007-40021. 2D-GE is unsurpassed in resolution (>5000 proteins), but suffers from lack of automation and reproducibility. Furthermore, proteins such as hydrophobic membrane proteins, basic proteins, acidic proteins, very large or very small proteins are poorly resolved.

For the many promising disease markers which escape identification using gel electrophoresis, 2D-LC provides a good alternative, allowing high-resolution separation of complex protein mixtures [Hanash et al. (2003) Nature 422, 226-232; Lipton et al. (2002) Proc. Natl. Acad. Sci. USA 99, 11049-11054]. 2D-LC approaches are more suitable than 2D-GE to separate smaller proteins and the automation and throughput is significantly better. Several technologies to separate protein/peptide digests by liquid chromatography have been described, including capillary electrophoresis (CE), reversed-phase (RP) HPLC, and multidimensional liquid chromatography.

Typically, peptides and proteins isolated by 2D-GE or 2D-LC are identified either by mass spectrometry or by determining amino acid composition/and or amino acid sequence.

The most relevant problem in determining statistically significant protein expression differences between different samples (e.g. disease vs. control) by mass spectrometry technologies is the relative quantitation of MS signals from different samples. Therefore, it is highly desirable to develop methodologies, which allow the processing of multiple samples in parallel in order to reduce the technical variability between different samples as much as possible.

### SUMMARY OF THE INVENTION

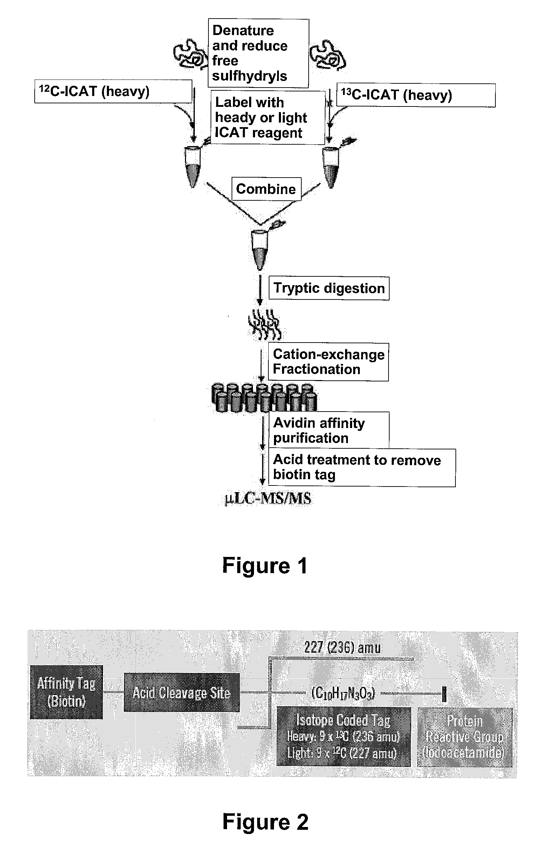

Recently, two approaches have been introduced to achieve this. The first technique is called Isotope-Coded Affinity Tag (ICAT) technology and allows for quantitative proteomic analysis based on differential isotopic tagging of related protein mixtures [Gygi et al. (1999) Nat. Biotechnol. 17, 994-999]. The ICAT reagent described uses three functional elements: a thiol-reactive group for the selective labelling of reduced cysteine residues, a biotin affinity tag to allow for selective isolation of cysteine labelled peptides, and an isotopic tag, which is synthesized in two isotopic forms, the "light" (non-isotopic) or "heavy" (utilizing ²H or ¹³C) form. As only a moderate number of generated peptides contains cysteine in its primary sequence, the complexity of the digested affinity purified sample decreases dramatically. Two individually labelled peptide samples ("light" vs. "heavy") are combined before they are applied to separation techniques like liquid chromatography (Figure 1) followed by MS identification.

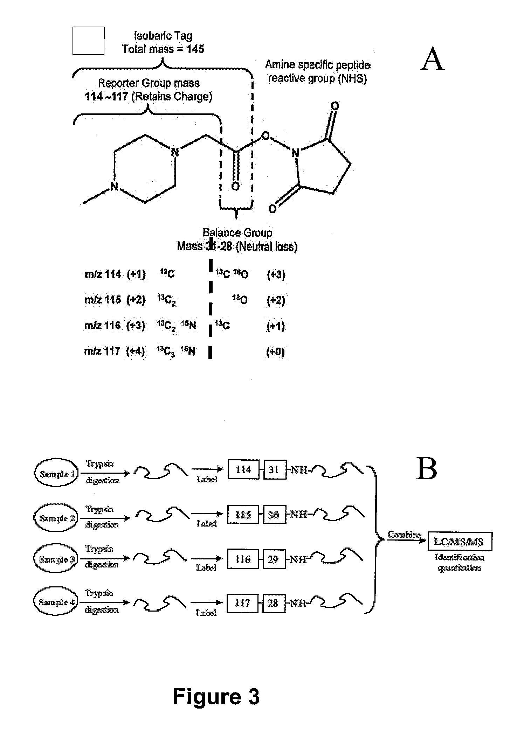

An alternative approach makes use of isobarically labelled reagents referred to as Isobaric Tag for Relative and Absolute Quantitation (iTRAQ). This technique described by Ross et al. (2004, Mol. Cell. Proteomics 3, 1154-1169) provides for four different iTRAQ reagents each containing a reporter group, a balance group and a peptide reactive group which reacts with primary amine groups (Figure 3). The reporter group is a group with a mass of 114, 115, 116 or 117 Da, depending on differential isotopic combinations of ¹²C/¹³C and ¹⁶O/¹⁸O in each reagent. The balance group varies in mass from 31 to 28 Da to ensure that the combined mass of the reporter group and the balance group remains constant (145 Da) for the four reagents. Accordingly, labelling of the same peptide with each of these reagents (comprising specific combinations of different isotopes in reporter and balance group) results in peptides which are isobaric and exactly co-elute in liquid chromatography and consequently are chromatographically indistinguishable from each other. During MS-MS analysis, the reporter group ions fragment from the peptides, displaying distinct masses of 114 to 117 Da. The intensity of these fragments can be used for quantitation of the individual peptides. Accordingly, the presence of a particular peptide in four different samples can be differentially determined by labelling each of the samples with a different iTRAQ reagent. iTRAQ labels are used to quantitate the relative as well as the absolute peptide abundance from MS/MS spectra and allows labelling of up to four different samples within a single experiment. In addition, the labelled peptides are isobaric and all contribute to one ion species that is observed in the MS and that is used for CID. This results in increased signal intensity and an increased probability of correct peptide identification, particularly for molecules which are not abundant, which is a characteristic of many biologically meaningful proteins. Recently, the set of commercially available ITRAQ reagents has grown to eight, including reporter groups with a Mass of 113, 118, 119 and 121.

Absolute and relative quantitation of MS signals is an unsolved issue in biomarker discovery by technologies based on mass spectrometry approaches. However, the identification of relevant expression differences between different samples, e.g., (different disease groups, different progression stages of a disease, disease vs. control/healthy) needs highly reproducible techniques, as relevant biomarkers can only be derived from a multitude of measurements.

The above described ICAT and iTRAQ techniques allow the multiplexing of either 2 (ICAT) or up to 4 or 8 (iTRAQ) individual samples and allows processing them simultaneously thereby minimising technical variability. Nevertheless, both these approaches have significant limitations. The ICAT approach only allows the investigation of two different samples, or two disease groups, which normally does not represent the full spectrum of disease relevant groups (e.g., different stages of progression of a disease). Therefore, it would be very advantageous to have a technique available, which allows the investigation of more than 2 groups. The iTRAQ technology, while allowing multiple sample analysis has the clear disadvantage that it requires performing MS/MS scans on each MS signal (for both unlabelled and labelled peptides) for relative quantitation of the reporter signals. In practice, this is not feasible when the starting material is of high complexity such as in human serum, or other body fluids, as the analysis will be very time consuming.

Particular and preferred aspects of the invention are set out in the accompanying independent and dependent claims. Features from the dependent claims may be combined with features of the independent claims and with features of other dependent claims as appropriate and not merely as explicitly set out in the claims.
The present invention overcomes the limited multiplexing of the prior art techniques by combining isotopic labelling and isobaric labelling.

The use of double labelling methods according to the present invention has the advantage that that more samples can be analysed in parallel while combining the strengths of both strategies, e.g. reduced sample complexity and increased sample through-put or decreased analysis-time.

The use of double labelling methods of the present invention have the advantage that the analysis time by mass spectrometry is significantly reduced, because only those peptides need to be analysed by MS/MS for which a differential expression of a peptide is observed between different samples.

The tools and methods making use of double labelling method of the present invention are of interest for multiple screening of protein samples. More particularly, they make it possible to simultaneously determine quantitative and/or qualitative differences of proteins in different protein samples. This can be used to determine differences in expression levels, and also allows the determining of differences in proteolytic processing between different protein samples, e.g. in the context of disease. In this regard the present invention provides methods for multiple screening of different samples which can be directly compared, e.g. in the comparison of different stages of a disease and/or different disease forms.

The present invention also allows the use of one label for an internal standard, which makes a run-to-run comparison feasible and makes absolute quantitation possible.

A first aspect of the invention relates to a method for simultaneously analysing the presence and/or quantity of one or more polypeptides comprising a first and a second functional group in different samples. This method comprises two labelling steps, wherein
- the first labelling step encompasses reacting a first set of labelling reagents with a first functional group of the one or more polypeptides in the samples, or peptides generated therefrom,
- the second labelling step encompasses reacting a second set of labelling reagents with a second functional group on the polypeptides in the samples, or peptides generated therefrom,
whereby one of the first and the second set of labelling reagents is a set of isobaric labelling reagents and the other is a set of isotopic labelling reagents, and wherein the combination is performed such that each of the samples is labelled with a different combination of the first and the second labelling reagents from the first and set second set of labelling reagents. Typically, the methods of the present invention further comprise the steps of pooling the different samples to obtain a polypeptide sample mix; selectively isolating the double-labelled polypeptides from the polypeptide sample mix, and analysing the relative occurrence of the isolated double-labelled polypeptides by Mass spectroscopy.

According to one embodiment of this method, the first set of labelling reagents is a set of isotopic labelling reagents and the second set of labelling reagents is a set of isobaric labelling reagents.

According to another embodiment of this method, the first set of labelling reagents comprises an affinity tag, which is introduced onto the polypeptide simultaneously with the label. Typically, the methods according to this embodiment of the invention include a step wherein the peptides are isolated based on the presence of the affinity tag.

Specific embodiments of the methods of the present invention include methods comprising a polypeptide cleavage step, to facilitate the analysis. According to a particular embodiment, this cleavage is performed with trypsin. According to one embodiment, the polypeptides present in the samples are cleaved into peptides after reaction with the first set of labelling reagents.

According to a particular embodiment of the methods of the present invention, the first labelling reagent is a reagent which reacts with a functional group which is a thiol and/or the second labelling reagent reacts with a functional group which is a primary amine.

According to particular embodiments the set of isotopic labelling reagents used in the methods of the present invention, has the general formula: 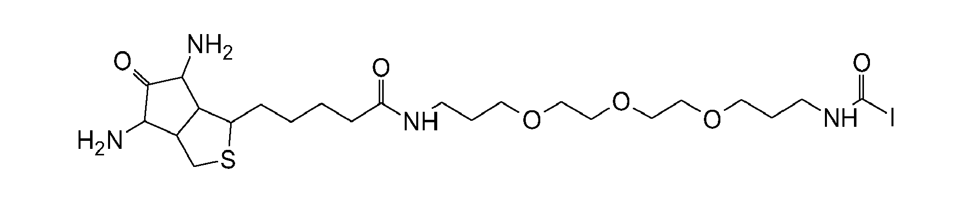 wherein one or more ¹³C isotopes are present in the linker between the biotin and iodoacetamide group.

According to a further particular embodiment of the methods of the present invention, the set of isobaric labelling reagents all comprise a molecule comprising a methyl piperazine derived moiety, a carbonyl moiety and a protein reactive group with the formula: 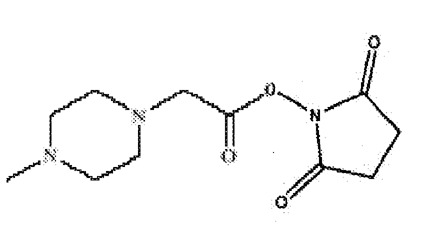 wherein one or more of the atoms in the methyl piperazine derived moiety or in the carbonyl moiety is an isotope selected from the group consisting of ¹⁸O, ²H, ¹⁵N and ¹³C.

According to a particular embodiment of the methods of the present invention, the first and/or second labelling reagents react with a first and/or second functional group of the polypeptides (or of the peptides generated therefrom). These functional groups are functional groups which are naturally present in the polypeptides or peptides or which have been introduced into by modification of another functional group. Accordingly, the methods of the present invention optionally comprise a step wherein the polypeptides or peptides present in the samples are subjected to one or more modifications so as to obtain one or more functional groups suitable for reacting with a protein reactive group of isotopic and/or isobaric labelling reagents.

According to a particular embodiment, the methods of the present invention further comprise the step of pooling polypeptides or peptides from two or more different samples after the first labelling step.

According to a particular embodiment of the methods of the present invention, the step of selectively isolating the double-labelled polypeptides from the polypeptide sample mix comprises the step of isolating a double-labelled polypeptide or peptide fraction from the pooled polypeptides by electrophoresis or chromatography.

According to a particular embodiment of the methods of the present invention, the step of analysing the relative occurrence of the isolated double-labelled polypeptides by Mass spectroscopy comprises the step of determining with MS the relative amount of different isotopes in the isolated double-labelled polypeptide or peptide fraction.

According to a particular embodiment of the methods of the present invention, the step of analysing the relative occurrence of the isolated double-labelled polypeptides or peptides by Mass spectroscopy comprises the step of subjecting the isolated double-labelled polypeptide or peptide fraction to collision-induced dissociation (CID) and determining the relative amount of different isobarically labelled polypeptides therein.

According to a particular embodiment of the methods of the present invention, the relative amount of different isobarically labelled polypeptide is determined by determining the relative amount of each of the reporter groups released from the isobaric label of each of the double-labelled polypeptides or peptides by CID.

Particular embodiments of the methods of the present invention include methods which further comprise the step of determining the sequence of the polypeptide.

A further aspect of the invention provides methods for double-labelling a polypeptide sample with one of a set of isobaric labelling reagents and one of a set of isotopic labelling reagents, which methods comprise the steps of reacting, in a first labelling step, a first labelling reagent with a first functional group on the polypeptides in the sample, and reacting, in a second labelling step, the second labelling reagent with a second functional group on the polypeptides in the sample. wherein one of the first and the second labelling reagent is an isobaric labelling reagent and the other is an isotopic labelling reagent.

Also envisaged within the scope of the present invention are methods as described above, wherein, instead of a set of isotopic labelling reagents, the isotopic labelling step is performed by labelling half of the samples by protease mediated ¹⁸O incorporation into the C-terminus of peptides of the polypeptides present in the sample, whereby the other half of the samples is treated with protease in the presence of ¹⁶O. According to this embodiment, methods for double labelling a sample with an isobaric and an isotopic label are provided, which methods comprise the following steps: (a) a first labelling step wherein at least one sample is isotopically labelled by protease mediated ¹⁸O incorporation into the C-terminus of the protease-generated peptides and (b) a second labelling step wherein a set of isobaric labelling reagents, is reacted with a functional group on the polypeptides in the samples, or peptides generated therefrom. In this embodiment, the combination of labelling steps is performed such that each of the samples is labelled with a different combination of O isotope and isobaric label. Besides the isotopic labelling step, the methods of double labelling according to this embodiment are essentially the same as described above. Accordingly, the methods according to this embodiment of the invention further comprise the steps of (c) pooling the different samples to obtain a polypeptide sample mix, (d) selectively isolating the double-labelled polypeptides from the polypeptide sample mix; and (e) analysing the relative occurrence of the isolated double-labelled polypeptides by Mass spectroscopy.

According to these embodiments of the methods of the present invention, the set of isobaric labelling reagents optionally comprises an affinity tag.

Further specific embodiments described for the double labelling methods above are also envisaged for double labelling methods whereby the isotopic labelling is ensured by protease-mediated ¹⁸O incorporation.

Accordingly, the present invention also encompasses methods for double-labelling a polypeptide sample with an isobaric labelling reagent and ¹⁸O comprising the steps of (a) incorporating in a first labelling step ¹⁸O in the C-terminus of proteolytic peptides of the polypeptides in the sample with a protease and H₂¹⁸O, and (b) reacting in a second labelling step an isobaric labelling reagent with a functional group on the polypeptides in the sample.

A further aspect of the invention relates to the use of the methods of double labelling as described above for identifying proteins which are differentially expressed between different protein samples such as, for instance a protein sample of a control individual and one or more samples from disease conditions, or in the comparision of different stages of a disease or the comparison of different experimental protein samples.

A further aspect of the present invention relates to polypeptides or peptides such as those obtained by the double labelling methods of the present invention, which polypeptides or peptides comprise one of a set of isotopic labels on a first functional group of the polypeptide and one of a set of isobaric labels on a second functional group of the polypeptide.

In one embodiment, in the above polypeptide one of either the isotopic or isobaric label is present on the side chain of an amino acid in the polypeptide and the other of the isotopic or isobaric label is present on the aminoterminus or the carboxyterminus of the polypeptide.

In particular embodiments of the polypeptides or peptides obtainable by the methods of the present invention, comprise an isobaric or isotopic label linked to a first functional group which is a thiol and a second isotopic or isobaric label, respectively, linked to a second functional group which is a primary amine. In specific embodiments, the thiol group to which the isotopic or isobaric label is linked is the thiol of a cysteine residue in the polypeptide or peptide and/or the primary amine to which the isotopic or isobaric label is linked is the primary amine group of the aminoterminus of the polypeptide or peptide.

In a particular embodiment, the double-labelled peptide is a tryptic peptide of a protein.

In another particular embodiment of the peptides obtainable by the methods of the present invention, one of the isotopic or isobaric labels present on the peptide or polypeptide is on the aminoterminus of the polypeptide and the other of the isotopic or isobaric labels is on the carboxyterminus of the polypeptide.

In a particular embodiment, the isotopic label on the polypeptide has a general structure:

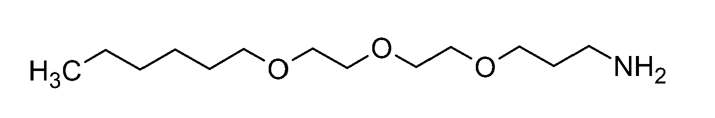

In an alternative embodiment, the isotopic label on the double-labelled peptide corresponds to the presence of one or two ¹⁸O or ¹⁶O incorporated into the C-terminus of the peptide.

Independently of the isotopic label, particular embodiments of the polypeptides and peptides of the invention comprise an isobaric label having the following general structure:

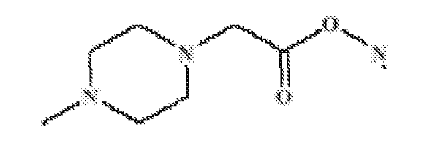

Yet a further aspect of the present invention relates to a mixture of polypeptides or peptides from different samples, this mixture comprising four or more polypeptides or peptides as described above, wherein polypeptides with an identical amino acid sequence from a different sample carry a different combination of an isotopic label and an isobaric label.

In a particular embodiment, the number of different isobaric labels present on the polypeptides or peptides within the mixture is 4 or 8 and the number of different isotopic labels is 2.

A specific embodiment provides mixtures of polypeptides with an identical amino acid sequence carrying different combinations of ¹⁸O isotopic or non isotopic ¹⁶O comprising C-termini and different isobaric labels.

Yet a further aspect of the present invention provides tools for the double labelling methods according to the present invention, such as, but not limited to kits of reagents comprising at least one reagent for isotopic labelling and one reagent for isobaric labelling, more spepcifically one set of at least two isotopic labelling reagents and one set of at least two isobaric labelling reagents, wherein the reagents have protein reactive groups.

In one embodiment, one of these sets of labelling reagents provided in the kit of reagents comprises an affinity tag.

Specific embodiments of the kits of the present invention provide kits of reagents comprising H₂¹⁸O and a set of at least two isobaric labelling reagents, wherein the reagents comprise a protein reactive group.

In one embodiment, the isobaric labelling reagents in the kit of reagents comprises an affinity tag.

A further aspect of the present invention provides methods of determining relative amounts of individual polypeptides or peptides of different samples with the same amino acid sequence present in a pooled mixture, wherein each individual polypeptide is double labelled with a different combination of one of a set of isotopic labels and one of a set of isobaric labels. The methods of this aspect of the invention comprise the steps of:
a) optionally isolating polypeptides or peptides by liquid chromatography, so as to obtain an isolated fraction comprising the individual polypeptides or peptides having the same amino acid sequence,
b) separating the isolated fraction of polypeptides or peptides obtained from step (a) into further fractions of polypeptides or peptides having a different mass by a first MS and determining the relative amount of each fraction with a different mass.
c) subjecting the peptide fractions obtained in step (b) to a second MS under conditions wherein the isobaric label (and optionally the polypeptide is fragmented) and determining the amounts of each fragmented isobaric label.
d) determining from the amount of the fragmented isobaric labels determined in step (c) the relative amount of the individual polypeptides or peptides within a peptide fraction obtained in step (b),
e) repeating steps (c) and (d) for each polypeptide or peptide fraction obtained in step (b)
f) calculating from the relative amount of each fraction with a different mass determined in step (b) and from the relative amounts of the individual peptides within a peptide fraction obtained in step (d), the relative amount of each double labelled polypeptide or peptide having the same amino acid sequence present in said pooled mixture.

A particular embodiment of this aspect of the invention provides methods as described above, wherein the peptides of different samples with the same amino acid sequence present in a pooled mixture are differentially double labelled with a different combination of one of a set of isotopic labels and one of a set of isobaric labels, whereby the isotopic label corresponds to the presence of one or two ¹⁸O or ¹⁶O incorporated into the C-terminus of peptides.

Yet a further aspect of the present invention relates to a device (100) for multiplex analysis of protein samples using double labelling comprising at least two sources of samples (101), two labelling units (103 and 103') and corresponding label sources (104 and 107) each comprising a set of reagents suitable for isotopic or isobaric labelling, a separation unit (108), a mass spectrometer unit (109) and a circuitry control and data analysis unit (110).

In one embodiment, the device further comprises one or more elements selected from the group consisting of a sample preparation unit (102), a protein cleavage unit (105) and an affinity purification unit (106).

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### BRIEF DESCRIPTION OF THE DRAWINGS

Fig. 1 shows a proteomic flow-chart for quantitative analysis of protein profiles with cleavable ICAT reagents (taken from Li et al. (2003) Mol. Cell. Proteom. 2, 1198-1204)
Fig. 2 shows a schematic structure of a cleavable ICAT reagent of the prior art.
Fig. 3 shows exemplary structures of iTRAQ reagents (top panel) and peptides labelled therewith (bottom panel). The reagent consisting of a reporter group with a mass ranging from 114 to 117 Da, a balance group with a mass ranging from 31 to 28 Da and an amine-specific peptide reactive group.

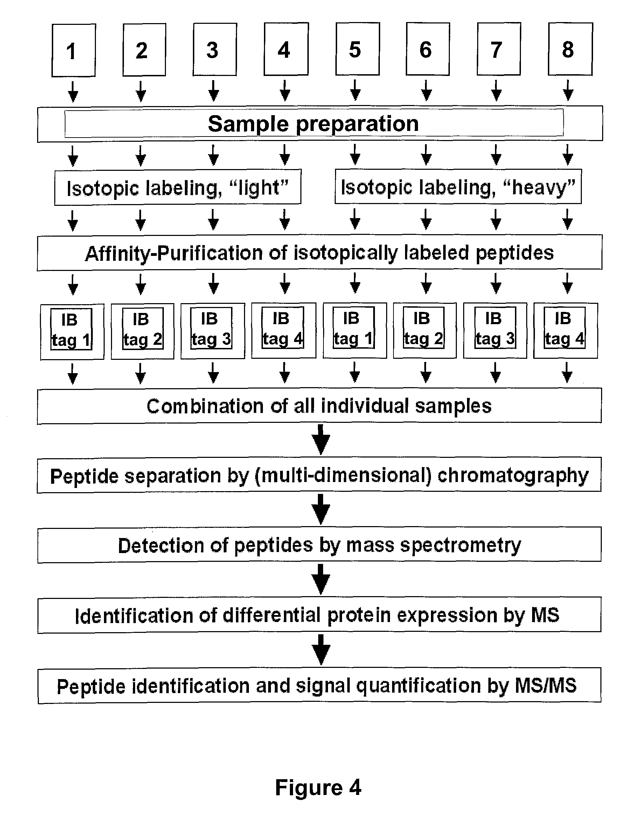

Fig. 4 demonstrates the simultaneous analysis of multiple samples (1 to 8) using double labelling with both isotopic and isobaric labels (IB) in accordance with particular embodiments of the present invention.
Fig. 5 method A: this shows a method as shown in Figure 4 wherein the first labelling is performed with the labelling reagent with the lowest complexity. Method B shows an alternative method wherein the first labelling is performed with the labelling reagent of the highest complexity.

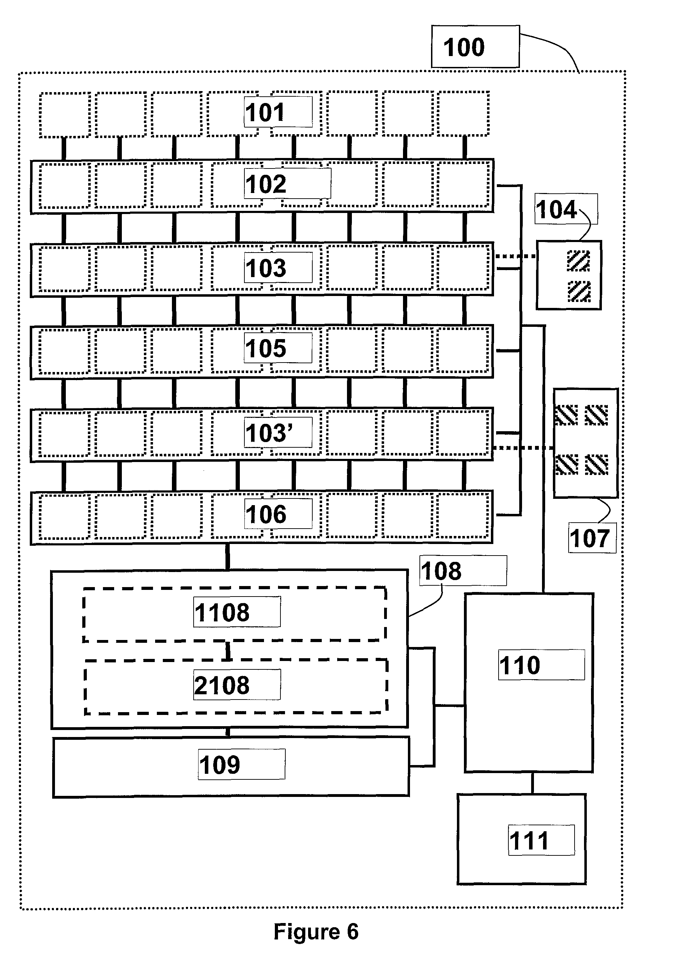

Fig. 6 shows in accordance with a particular embodiment of the present invention a device for multiplex analysis of 8 protein samples using double labelling (100), comprising at least two sample sources (101), a sample preparation unit (102), a first labelling unit (103) with at least two first labelling reagent sources (104), a protein cleavage unit (105) an affinity-purification unit (106) (e.g. avidin affinity chromatography system), a second labelling unit (103') with at least two (here four) second labelling reagent sources (107), a separation unit (108) comprising two consecutively coupled separation systems (1108) and (2108), a mass spectrometer unit (109) and a control and analysis circuitry and data analysis unit (110) coupled to a read out system (111).

In the different Figures, the same reference signs refer to the same or analogous elements.

### DETAILED DESCRIPTION OF THE EMBODIMENTS

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. These definitions should not be construed to have a scope less than understood by a person of ordinary skill in the art.

The term "polypeptide" or "protein", as used herein, refers to a plurality of natural or modified amino acids connected via a peptide bond. The length of a polypeptide can vary from 2 to several thousand amino acids (the term thus also includes what is generally referred to as oligopeptides). Included within this scope are polypeptides comprising one or more amino acids which are modified by in-vivo posttranslational modifications such as glycosylation, phosphorylation, etc. and/or comprising one or more amino acids which have been modified in-vitro with protein modifying agents (e.g. alkylating agents).

The term "polypeptide fragment" or "peptide" as used herein is used to refer to the amino acid sequence obtained after cleavage of a protein or polypeptide. A polypeptide fragment or peptide is not limited in size or nature.

The terms "internal", "aminoterminal" and "carboxyterminal" when referring to a peptide are used herein to refer to the corresponding location of a peptide in a protein or polypeptide. For example, in a tryptic cleavage of protein NH₂-X₁-K-X₂-R-X₃-K-X₄-COOH (wherein X₁, X₂, X₃ and X₄ are peptide sequences of indifferent length without Lysine or Arginine), the aminoterminal peptide is NH₂-X₁-K-COOH, of the internal peptides are NH₂-X₂-R-COOH and NH₂-X₃-K-COOH and the carboxyterminal peptide is NH₂-X₄-COOH.

The term "protein cleavage" as used herein relates to the hydrolysis of a peptide bond between two amino acids in a polypeptide. This includes both chemical or enzymatic hydrolysis.

The term "fragmentation" as used herein refers to the breaking of one or more chemical bonds and subsequent release of one or more parts of a molecule as obtained e.g. by collision-induced dissociation (CID) in tandem Mass spectrometry (MS) or MS/MS analysis. In certain embodiments the bond is a peptide bond, but it is not limited thereto.

The term "label" as used herein refers to a compound or molecule, which can be covalently linked to or incorporated in a peptide or polypeptide and which, based on its particular properties is detectable on a mass spectrometer. Where the label can be covalently bound to a peptide or polypeptide, this is ensured by a protein/peptide reactive group, present in the labelling reagent. The term label is used herein to generally refer to both label (e.g. as bound to a protein or peptide) and labelling reagent (more specifically referring to the nature of the molecules prior to the binding with the peptide or protein). The present invention envisages the use of different types of labels, such as isotopic and isobaric labels defined below. The term 'set of labels' as used herein with regard to either isotopic or isobaric labels, refers to different labels of one type which can be used simultaneously in one experiment to label different samples, i.e. which have the same chemical structure but can be differentiated based on mass in MS or MS/MS.

The term "isotopic labels" as used herein refers to a set of labels having the same chemical formula but differing from each other in the number and/or type of isotopes present of one or more atoms, resulting in a difference in mass on MS. Thus, identical peptides labelled with different isotopic labels can be differentiated as such on MS based on difference in mass. While isobaric labels (see below) in principle constitute a specific type of isotopic labels, in the context of the present invention, the term isotopic label will be used to refer to labels which are not isobaric, but can as such be differentiated based on their molecular weight without fragmentation.

The term "isobaric labels" as used herein refers to a set of labels having the same structure, and the same mass, which upon fragmentation release a particular fragment with the same structure for all isobaric labels of that set, which differs in mass between the individual isobaric labels in that set, due to a differential distribution of isotopes within the isobaric labels. Isobaric labels typically comprise a reporter group (RG), which is a relatively small fragment and a balance group (BG). The "combined mass" of a set of isobaric labels refers to the total mass of the reporter group and the balance group for that set of isobaric labels.

The term "reporter group (RG)" refers to the part of isobaric labels which generates a strong signature ion upon Collision Induced Dissociation (CID). The typical fragments generated upon release of the reporter group are used to quantitate an isobaric-labelled polypeptide. Typically the fragment ions appear in the low-mass region of an MS spectrum, where other fragment ions are not generally found.

The term "balance group (BG)" as used herein refers to the part of isobaric labels, which contains a certain compensating number of isotopes so as to ensure that the combined mass of the reporter group and balance group is constant for the different isobaric labels. The balance group may or may not be released from the label upon CID.

The term "protein/peptide reactive group" (PRG) as used herein refers to a chemical function on a compound that is capable of reacting with a functional group on an amino acid of a protein or peptide resulting in the binding (non-covalent or covalent) of such compound to the amino acid.

The term "functional group" as used herein refers to a chemical function on an amino acid which can be used for binding (generally, covalent binding) to a chemical compound. Functional groups can be present on the side chain of an amino acid or on the aminoterminus or carboxyterminus of a polypeptide or peptide. The term encompasses both functional groups which are naturally present on a peptide or polypeptide and those introduced via e.g. a chemical reaction using protein-modifying agents.

The present invention relates to the combined use of different labelling reagents which react with polypeptides and/or peptides and which allow differentiation of similar peptides of different origin within one pooled polypeptide or peptide mixture and to identify the presence and the relative amount of a protein or a peptide in a pooled sample. More particularly, the present invention relates to the combined use of isotopic labels and isobaric labels for multiplexed protein analysis.

Accordingly, the methods and tools of the present invention are of particular interest in the analysis of a set of samples for which a comparable analysis is of interest. Such a set of samples can be, but is not limited to, samples from a patient taken at different time points, samples of different clinical versions of a disease, samples of different patients etc..

The methods and tools of the present invention relate to the analysis of protein samples. The term 'sample' as used herein is not intended to necessarily include or exclude any processing steps prior to the performing of the methods of the invention. The samples can be rough unprocessed samples, extracted protein fractions, purified protein fractions etc... As indicated above, the proteins present in the samples. According to one embodiment the protein samples are pre-processed by immunodepletion of abundant proteins.

Protein samples which are suitable for analysis with the labelling reagents and methods of the present invention include samples of viral, prokaryote, bacterial, eukaryote, fungal, yeast, vegetal, invertebrate, vertebrate, mammalian and human origin.

The preparation of samples differs depending on the organism, tissue or organ investigated, but standard procedures are usually available and known to the expert. With respect to mammalian and human protein samples it covers the isolation of cultured cells, laser micro-dissected cells, body tissue, body fluids, or other relevant samples of interest. With respect to the fractionation of proteins in a sample, cell lysis is the first step in cell fractionation and protein purification. Many techniques are available for the disruption of cells, including physical, enzymatic and detergent-based methods. Historically, physical lysis has been the method of choice for cell disruption; (homogenisation, osmotic lysis, ultrasound cell disruption) however, it often requires expensive, cumbersome equipment and involves protocols that are sometimes difficult to repeat due to variability in the apparatus (such as loose-fitting compared with tight-fitting homogenization pestles). In recent years, detergent-based lysis has become very popular due to ease of use, low cost and efficient protocols.

Mammalian cells have a plasma membrane, a protein-lipid bilayer that forms a barrier separating cell contents from the extracellular environment. Lipids comprising the plasma membrane are amphipathic, having hydrophilic and hydrophobic moieties that associate spontaneously to form a closed bimolecular sheet. Membrane proteins are embedded in the lipid bilayer, held in place by one or more domains spanning the hydrophobic core. In addition, peripheral proteins bind the inner or outer surface of the bilayer through interactions with integral membrane proteins or with polar lipid head groups. The nature of the lipid and protein content varies with cell type. Clearly, the technique chosen for the disruption of cells, whether physical or detergent-based, must take into consideration the origin of the cells or tissues being examined and the inherent ease or difficulty in disrupting their outer layer(s). In addition, the method must be compatible with the amount of material to be processed and the intended downstream applications.

In particular embodiments, protein extraction also includes the pre-fractionation of cellular proteins originated from different compartments (such as extracellular proteins, membrane proteins, cytosolic proteins, nuclear proteins, mitochondrial proteins). Other pre-fractionation methods separate proteins on physical properties such as isoelectric point, charge and molecular weight.

According to a particular embodiment, the samples are pre-treated prior to labelling or cleavage, so as to denature the proteins for optimised access to reagents or proteases, using appropriate agents (e.g., guanidinium chloride, urea, acids (e.g. 0,1 % trifluoric acid), bases (e.g. 50 % pyridine) and ionic or non-ionic detergents).

Furthermore, as will be detailed below, depending on the type of protein reactive groups in the labelling agent, the methods of the present invention envisage that the functional groups are irreversibly or reversibly modified with protein modification agents, prior to the double labelling methods of the invention. Examples are the release of blocked aminotermini, the reduction of cystine to cysteine, blocking the functional thiol group of cysteine, alkylating the amine group of lysine.

The methods of the present invention thus optionally comprise a pre-treatment step, which can be performed in a pre-treatment step comprising one or more of the sample preparation methods listed above. Accordingly, devices suitable for the methods of the present invention optionally comprise a sample preparation unit comprising one or more devices suitable for sample preparation e.g. sonication devices, chromatography systems (affinity, gelfiltration), ultrafiltration units, centrifuges, temperature controlled reaction vials with delivery systems for buffers, enzymes, detergents etc...

In the methods of the present invention two labelling steps are performed. According to one embodiment, the methods of the invention comprise the treatment of each of a set of different samples with a unique combination of an isotopic and an isobaric labelling reagent.

According to a particular embodiment, the method comprises the steps of labelling proteins present in a set samples with a first set of labels on a first functional group of the proteins, thereafter, cleaving the labelled proteins into peptides, and labelling the labelled proteins with a second set of labels on a second functional group of the proteins. In both labelling steps, care is taken to ensure different combinations of first and second label for each of the samples of the set. Typically, this is performed in such a way to ensure a maximal number of different combinations. Where the number of labelling reagents available for the first labelling reagent is X, and the number of labelling reagents available for the second labelling reagent is Y, this is ensured by a) dividing the number of samples into X, and labelling each of these groups of samples with a different first labelling reagent and b) within each of these groups labelling each of the samples with a different second labelling reagent.

In the steps following the labelling, the double-labelled peptides are isolated and analysed for each of the samples.

In a particular embodiment of the methods of the invention, the first and the second set of labels are either isotopic or isobaric labels. Whether an isobaric or isotopic label is used as first label depends from the experimental set-up.

According to a particular embodiment, the first set of labels is a set of isotopic labels comprising an affinity tag. According to this embodiment, the labelled proteins are cleaved and after cleaving, the labelled peptides are isolated with the affinity tag, which is present in the label. This affinity isolation allows for a reduction of the complexity of the sample for further labelling and analysis. When a protein cleavage is performed, peptides which have no first functional groups and which are not labelled are removed.

In the methods of the present invention one or more proteins present in or isolated from a sample are labelled by reacting a functional group present on these one or more proteins with a protein reactive group in a labelling reagent. Ideally, the labelling targets almost every protein in the sample to obtain a maximal coverage of the proteome in a sample.

At the same time, when considering each individual protein, the number of functional groups in that protein is ideally low, so as to reduce the complexity of the analysis (number of peptides to be analysed from this protein in a sample is only one or a limited number).

Cysteine is often used as functional group for the first labelling step because cysteine labelling provides a compromise between maximal coverage (labelling every protein in a sample) and minimal complexity (labelling every protein in a sample only once or a limited number of times). Cysteine is present in about 85 % of all proteins in Genbank and on average occurs with a frequency of 2 % (one in each 50 amino acids) in a polypeptide sequence. Furthermore, labelling with cysteine reactive reagents does not modify other parts of a protein as is the case with the labelling amine groups which occur on both Lysine and the aminoterminus or as is the case with labelling carboxyl groups which occur on both Aspartic acid, Glutamic acid and the carboxyterminus. According to one embodiment of the methods of the invention, the first labelling step is thus performed through a cysteine functional group. In a particular embodiment, prior to the first labelling step, the samples are reduced to ensure the presence of an accessible thiol functional group on the cysteines in the proteins of the sample.

Moreover, the chance that cysteine appears in either the C-terminal peptide or the N-terminal peptide of a given enzymatic digest decreases with the number of recognition sites for such enzyme in a protein. This number normally increases proportionally with the length of a polypeptide. Thus the double-labelling methods wherein cysteines is used as the functional group of the first labelling reagent are very well suited for determining relative expression levels of a protein in a sample. The technique is less suited for assays wherein enzymatic processing of a sample is studied (so called degradomics, see below).

For the second labelling, it is the aim to label herein every peptide which has been labelled in the first labelling step (and optionally isolated thereafter). When the proteins are digested after the first labelling, each internal peptide from the digest achieves a novel amino group and a novel carboxyterminal group. From these two, the aminoterminus is the easiest to modify with protein reactive groups such as NBS. Apart from the primary amine group on the N-terminus, the amine group on the epsilon position of lysine will also be labelled. Where this is undesirable, an additional step is envisaged to block these groups prior to the protein cleavage. Similarly, in particular embodiments of the methods of the invention, where the second labelling is performed with a carboxyl reactive labelling reagent, it is envisaged that an additional step can be included so as to block carboxyl groups of Asp and Glu prior to the protein cleavage.

Generally analysis methods based on MS involve a cleaving step. This is generally performed to allow the analysis of smaller peptides, rather than the full-length proteins. It is generally easier to separate peptides than proteins on high throughput systems such as LC, and to interpret sequence data from peptides in MS/MS. However, the use of the labelling methods of the present invention is also envisaged in analysis methods that do not include a cleavage step. Full length polypeptides can be labelled. Additionally, where the compound of interest is a natural peptide, labelling can be performed directly. According to a particular embodiment, as indicated above, the methods of the invention comprise a cleaving step, whereby the cleaving step can be either prior to the first labelling step or between the first and the second labelling step.

Suitable chemicals for protein cleavage include cyanogen bromide, BNPS skatole (2-(2'-Nitrophenylsulfonyl)-3-methyl-3-Bromoindolenine), formic acid, hydroxylamine, iodobenzoic acid, NTCB + Ni (2 nitro 5 thiocyanobenzoid acid). Suitable proteolytic enzymes include Asp-N Endopeptidase, Caspase 1, 2, 3, 4, 5, 6, 7, 8, 9, or 10, Chymotrypsin; Clostripain, Enterokinase, Factor Xa, Glutamyl Endopeptidase, Granzyme B, LysC Lysyl endopeptidase, Papain, Pepsin, Proline-Endopeptidase, Proteinase K, Staphylococal peptidase I, Thermolysin, Thrombin, Trypsin.

Depending on the type of protein sample a combination of chemical and enzymatic cleavage is performed or a double enzymatic digestion is performed.

Despite the variety in enzymes and chemicals, trypsin still remains the enzyme with the highest specificity (Lysine and Arginine) and efficiency. In vertebrates Lysine occurs in proteins with a frequency of 7,4 % and Arginine with a frequency of 4,2 %. A tryptic digest results thus in peptides with an average length of 9 amino acids on non-modified proteins and of 25 amino acids when lysine is modified to an extent where it is no substrate anymore for trypsin. Such peptides are well suited for chromatographic and MS techniques.

According to a particular embodiment of the methods of the invention, the cleavage of proteins is performed after the first labelling. This allows to reduce the complexity of a sample by isolating only those peptides which carry a label on a functional group.

According to a particular embodiment labelled proteins are digested with trypsin. According to a more particular embodiment labelled proteins are treated with an acetylating agent to modify the side chains of lysine before a tryptic digestion is performed. As a consequence the aminoterminus of a polypeptide will be acetylated equally.

According to another particular embodiment, labelled proteins are digested with a lysine specific protease. As a result all peptides obtained from such a digest will have an amine group at their aminoterminus and at the side chain of the carboxyterminal lysine (with the exception of the carboxyterminal peptide which may have another carboxyterminal amino acid). Proteins which are labelled in the first labelling step but which do not comprise a protein cleavage site can participate in the second labelling step as cleaved peptides. For example, when the first labelling is Cysteine specific and the second labelling is performed at the N-terminus, uncleaved Cysteine containing peptides with a accessible N-terminus will be also labelled.

In one aspect of the present invention, the double labelling is used to allow the simultaneous analysis of different samples. Accordingly, specific embodiments of the methods of the present invention comprise a step of pooling two or more samples which are differentially labelled in the first and second labelling step described above. This allows the immediate comparison of the different samples in the analysis. Where the protein samples used in the methods of the invention are complex, the pooled sample will be subjected to one or more peptide separation techniques. According to the present invention, the chemical structure of the labels within both the first and the second set of labels is the same, such that it will not generate a significant difference in properties in (multi-dimensional) chromatography techniques, between identical peptides that are differently labelled.

Suitable separation techniques, which allow the separation of a complex protein or peptide sample into two or more, up to multiple fractions (in the context of the isolation of double-labelled peptides) are known to the skilled person and include, but are not limited to isoelectric focusing, SDS PAGE, 2-dimensional gel electrophoresis, size-exclusion chromatography, ion exchange chromatography, reversed-phase HPLC, affinity chromatography, ... etc.

When the sample consists of larger proteins or peptides (e.g. between Mr 3000 and 100.000) 2D GE can be used. For peptide samples, obtained from e.g. proteolytic digestions, 2D LC approaches are more suitable for separation, and also the automation and throughput is significantly better. Several technologies to separate protein/peptide digests by liquid chromatography have been described, reversed-phase (RP)-HPLC, and 2-dimensional liquid chromatography. Also capillary electrophoresis (CE) is a method suitable for the separation peptides.

2D-LC generally uses ion-exchange columns (usually, strong cation exchange, SCX) on-line coupled with a reversed phase column, operated in a series of cycles. In each cycle the salt concentration is increased in the ion-exchange column, in order to elute peptides according to their ionic charge into the reversed phase system. Herein the peptides are separated on hydrophobicity by e.g. a gradient with CH₃CN.

Many parameters influence the resolution power and subsequently the number of proteins that can be displayed by LC-MS. Usually, the 'on-line' configuration between the first-dimension separation technique (SCX) and the second-dimension RP-HPLC separation approach is set up for sample fractionation. Ion exchange chromatography can be performed by stepwise elution with increasing salt concentration or by a gradient of salt. Typically, SCX is performed in the presence of, e.g. up to 30% acetonitrile, to minimize hydrophobic interactions during SCX chromatography. Prior to Reversed Phase chromatography on e.g. a C18 column, organic solvents such as acetontile are removed, or strongly reduced by e.g. evaporation.

Accordingly devices suitable for performing the methods of the present invention optionally contain or are connected to one or more suitable separation instruments, such as electrophoresis instruments, chromatography instruments, such as, but not limited to capillary electrophoresis (CE) instruments, reversed-phase (RP)-HPLC instruments, and/or 2-dimensional liquid chromatography instruments,... etc.

The present invention provides tools and methods for the simultaneous identification (by MS/MS) and quantitation (by MS or MS/MS) of proteins in different samples. More particularly, the methods of the present invention relate to the identification and quantitation of peptides from different samples in MS and MS/MS using differential labelling. Accordingly, the devices for performing the methods of the present invention comprise one or more mass spectrometric instruments.

Mass measurements by spectrometry are performed by the ionization of analytes into the gas phase. A typical mass spectrometric instrument consists of 3 components, an ion source to generate ions from the molecules of interest, a mass analyzer, which determines the mass-to-charge ratio (m/z) of the ionized molecules, and a detector that registers and counts the number of ions for each individual m/z value. Each feature in an MS spectrum is defined by two values, m/z and a measure on the number of ions, which reached the detector of the instrument.

The ionization of proteins or peptides for mass analysis in a spectrometer is usually performed by Electro-spray ionization (ESI) or matrix-assisted laser desorption/ionisation (MALDI).

During the ESI process analtyes are directly ionized out of solution and ESI is therefore often directly coupled to liquid- chromatographic separation tools (e.g., reversed phase HPLC). MALDI vaporizes via laser pulses dry samples mixed with small organic molecules that absorb the laser energy like cinnamic acid to make the process more effective.

The mass analyser is a key component of the mass spectrometer; important parameters are sensitivity, resolution, and mass accuracy. There are five basic types of mass analysers currently used in proteomics. These include the ion trap, time-of-flight (TOF), quadrupole, Orbitrap, and Fourier transform ion cyclotron (FTICR-MS) analysers. Tandem MS or MS/MS can be performed in time (ion trap) and in place (with all hybrid instruments such as e.g. LTQ-FTICR, LTQ-Orbitrap, Q-TOF, TOF-TOF, triple quad and hybrid triple quadrupole/linear ion trap (QTRAP))

As detailed herein, the spectrum generated on a mass spectrometer of a polypeptide or peptide which has been previously isolated from a pool of differentially labelled samples, contains a set of peaks with the characteristic mass differences between the different isotopic labels (the number of peaks will be less if a protein wherein this polypeptide occurs is not expressed at all in one of the samples). Each of these isotopically labelled peptides are further analyzed by tandem mass spectrometry to release the reporter group of the isobaric label for identification and further quantitation of the differently labelled peptides. Based on Mr of the polypeptide, the amino acid composition or the amino acid sequence, the identity of the individual peptides is determined.

Accordingly one aspect of the invention relates to a device for multiplex analysis of protein samples using double labelling (100) comprising at least two sample sources (101), labelling units (103) and (103'), with corresponding first and second label sources (104 and 107), a separation unit (108), a mass spectrometer unit (109) and a control circuitry and data analysis unit (110). In particular embodiments separation unit 108 comprises two consecutively linked separation systems 1108 and 2108, wherein e.g. 1108 is a 2-D gel electrophoresis system or a cation exchange chromatography system and separation system 2108 is typically a HPLC reversed-phase system.

Mass spectrometer element 109 is an MS/MS spectrometer which separates isotopic forms, wherein, upon CID, the reporter groups of the isobaric labels are differentially detected and wherein de novo peptide sequencing can be performed. MS/MS analysis can be done using 2 fundamentally different instruments. In the first type of instrument, the ion trap in which MS/MS analysis is done in the same ion trap where MS is performed, but MS/MS is done in time (trap is filled, all ions are ejected except ion(s) of interest and CID is performed and the fragment ions are scanned. The second type of instruments, hybrid instruments (triple quad, q-tof, ltq-ftms, ltq-orbitrap), separate MS/MS in place. e.g. parent selection is done in the first mass analyser and fragments are scanned in the second mass analyser.

The device can further comprise a number of optional elements such as a sample preparation unit (102) wherein one or more sample preparation methods such as but not limited to sample lysis and immunodepletion take place, a protein cleavage unit (105) and an affinity purification unit (106) to selectively isolate labelled polypeptides carrying an affinity tag (e.g. biotin - avidin affinity purification). Optionally, the protein cleavage unit is part of the sample preparation unit.

The methods of the present invention are hereafter illustrated by a number of labelling schemes.

As will become clear from the following different illustrative labelling schemes, the fragmentation of peptides or the use of an affinity tag is not compulsory for all embodiments of the present invention. Equally, it is possible that the first and second functional group in the two labelling steps are the same.

The following labelling scheme (see figure 3) discloses an embodiment of a double labelling of 8 protein samples using the two commercially available ICAT isotopic reagents reactive with cysteine and the four commercially available iTRAQ isobaric labelling reagents reactive with primary amines (reagents available from Applied Biosystems). The individual labelling steps are performed using the instructions as supplied with the labelling reagents.

In a pre-processing step, the samples are reduced to ensure the presence of an accessible thiol functional group on cysteine. According to this particular embodiment the samples are also acetylated to block the amines of the side chain of lysine. According to alternative embodiments this acetylation step is left out.

The different steps of the labelling scheme comprise:
- The 8 protein samples are each labelled with biotin tagged cysteine reactive ICAT isotopic labelling reagents, whereby 4 samples are reacted with one isotopic label ("light") and 4 samples with the other isotopic label ("heavy").
- The cysteine labelled samples so obtained are then digested with trypsin.
- To reduce the number of peptides for further analysis, the ICAT labelled peptides are isolated from each of the samples via the biotin affinity tag present in the label using (strept)avidin affinity techniques.
- In each sample, the isolated isotopic labelled peptides so obtained are labelled at the aminoterminus using one of the four amine reactive iTRAQ isobaric labels, so as to obtain eight different combinations of isotopic and isobaric labels. Optionally, one of the iTRAQ positions is used for an internal standard.

After the second labelling step each sample now comprises peptides with a specific combination of an isotopic label and an isobaric label (with the exception of aminoterminal peptides with cysteine which have a blocked aminoterminus). The method further includes the steps wherein:
- The double-labelled peptides obtained in the previous step from all samples are pooled.
- The pooled peptides are separated by one or more separation techniques such as but not limited to liquid chromatography. Herein peptides that are identical but which originate from a different sample and are thus differently double-labelled will behave in an essentially identical manner. Despite the difference in the combination of isotopic and isobaric label which is present on the peptide will not affect their elution as the different labels in each label set have the same chemical formula.
- Each separated peptide fraction is analysed by MS, which should generate two signals, with a different mass, corresponding to the presence of a "light" or a "heavy" isotopic label. Typically, when using existing isotopic labelling reagents, there is a resulting mass difference of 9 Da. Hereafter, each peptide with a different isotope is further fragmented whereby the reporter group is released from the isobaric label present on each of the peptides. The relative amount of reporter (with Mr 114, 115, 116, 117 using commercially available iTRAQ reagents) indicates the relative amount of peptide which was labelled with that reporter. MS data are further used to determine the sequence of the peptide and to identify the protein it is derived from. A theoretical example of determining the relative amount of each labelled peptide in a peptide mixture is shown in example 1 herein.

The method of the invention as illustrated above with commercially available labelling reagents allows multiplexing of different protein samples up to a complexity of 8 or 16. When additional isobaric and/or isotopic labels are used, this allows for increased multiplexing. The multiplexicity is determined by the product of the number of different labels of the first set of labels and the number of different labels in the second set of labels. More particularly, it is determined by the number of different isotopic labels times the number of different isobaric labels that can be used simultaneously.

The above-described exemplary labelling protocol is suitable for determining e.g., differences in expression level in-between individual proteins in different samples using internal peptides. Different alternative protocols are envisaged, depending on particular applications.

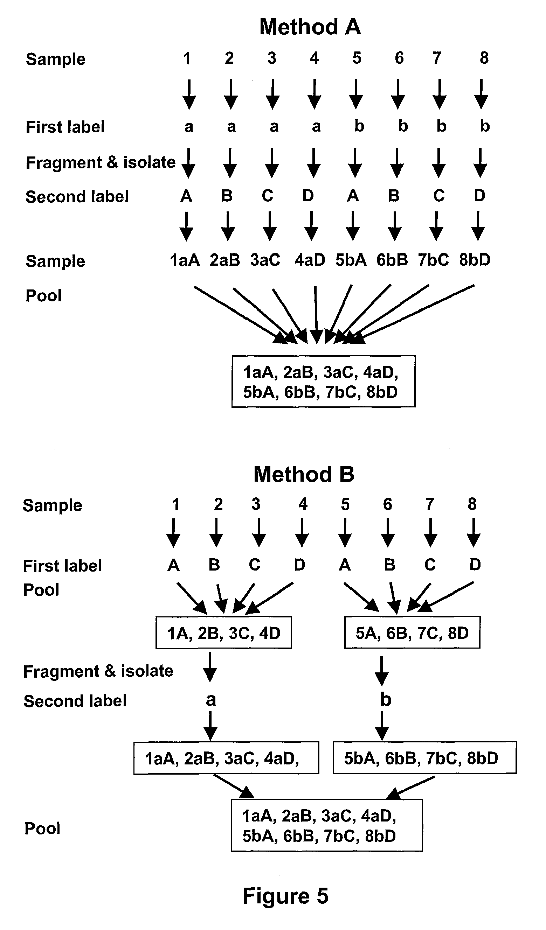

In the above outline protocol, all samples are labelled separately, and pooled after the second labelling step. In this particular embodiment, this implies eight separate digestions, eight different affinity purifications and eight different second labellings. However, to avoid differences between the samples due to technical manipulations, it is desirable that as many steps as possible are performed on pooled samples. Accordingly, in one embodiment of the invention the first labelling is performed with an (affinity-) tagged labelling reagent which has the highest complexity. Thus, according to a particular embodiment, the first labelling is performed with a set of four isobaric affinity tagged labelling reagents, while the second labelling is performed using two isotopic untagged labels. This method is outlined in Figure 5 wherein the method as described above (Method A in Figure 5 is compared with a method wherein four isobaric cysteine-reactive tagged reagents are used in the first labelling step and two untagged amine reactive reagents are used in the second labelling step (Method B in Figure 5). Where the complexity of the first labelling agent is 4 and the second labelling agent is 2, this allows pooling of the samples in two batches after the first labelling and performing digest, affinity separation and second labelling on all of the samples present in each of the two batches together. As detailed above, similar methods with other combinations of labelling reagents are also envisaged within the context of the present application.

A second exemplary double-labelling scheme according to the methods of the present invention is one that is considered particularly suitable e.g. to study proteolytic processing of proteins (so-called degradomics) in samples. In this embodiment, the double labelling makes use of the primary amine groups and carboxyl groups of the protein or peptides as functional groups. The methods of the invention differ depending on whether aminoterminal processing or carboxyterminal processing is to be considered.

For the investigation of aminoterminal processing, the different samples are first labelled at the primary amine group of the N-terminal amino acid of the proteins using a labelling reagent targeting these primary amine groups and further comprising an affinity tag. (optionally, the labelling is preceded by methods which release blocked aminoterminal groups (e.g. formyl groups)). It is noted that this first labelling step will also label all lysine side chains. In the next step, the proteins in the sample are for example digested with trypsin and the labelled peptides are isolated using the affinity tag. This isolate thus contains aminoterminal peptides and peptides with lysine. Typically, the peptides of interest (the aminoterminal peptides) will only represent a minority of the peptides in the isolate due to the frequent occurrence of Lysine in proteins (7 % in proteins of vertebrates). However, as a result of the digest, all peptides in the sample now have a novel free primary amine group except for the N-terminal peptide of each protein, which was already labelled). Using a second affinity separation with e.g. an amine reactive group on a column or on magnetic beads, all of these peptides are removed from each sample, except for the N-terminal peptide of interest. In particular embodiments it is possible to perform the amine affinity chromatography as sole affinity purification step after the protein digest. In this case it is possible to use a first labelling reagent without a tag.

After the first labelling and the affinity purification, a second labelling is performed on the isolated N-terminal peptides comprising the first label, with a label which is reactive against a carboxyl group. In this second labelling step, the carboxyterminus is labelled as well as the side chains of Asp and Glu, if these are not purposely modified in a blocking step prior to the labelling procedure.

Where the methods of the present invention are applied in the field of degradomics, e.g. to determine the effect of a specific enzyme, more particularly on the generation of small peptides and/or proteins, the prior modification of Asp and Glu in the intact proteins may not need to be performed. In these circumstances, the chances that a peptide is effectively digested by that specific enzyme is low. Consequently, in most cases, no novel carboxyterminus is generated and the protein can not be labelled with a second label. In particular embodiments, where the degradome of short proteins is studied a sample is first fractionated on Mr whereafter the first aminoterminal labelling and the second carboxyterminal labelling are performed without modification of COOH groups.

Alternatively when the processing of the carboxyterminus is studied an analogous method is performed wherein proteins are first labelled at the carboxyterminus, optionally amine groups are blocked, proteins are cleaved, carboxyterminal peptides are isolated and labelled at the newly created aminoterminus.

In a particular embodiment of the tools and methods of the present invention, the functional group for the first labelling reagent and the functional group for the second labelling reagent are the same. For example, the first labelling reagent reacts with the amine group of lysine. Hereafter, the protein is cleaved and labelled peptides are isolated. These peptides have a free aminoterminus which reacts with a second labelling reagent which also has a protein reactive group reacting with amines. In this embodiment an aminoterminal peptide, which is labelled only with the first label, will also end up in the peptide mixture which is analysed with MS/MS.

The most conventional method as outlined above uses a first labelling on a side chain of a peptide followed by a second labelling at the aminoterminus of the already labelled peptides. Typically the labelling on the side chain is performed on cysteine. However as mentioned before about 15 % of proteins in the protein database have no cysteine and will not be labelled. The chance that no cysteine occurs in a protein increases with decreasing protein length. This will be equally true for the labelling at side chains of amino acids other than cysteine. The shorter the protein, the smaller the chance for labelling on the side chain of an amino acid.

To study low Mr proteins, double labelling methods of the present invention using primary amines and on carboxyl groups as functional groups are of particular interest, as these groups always occur in each protein at respectively the aminoterminus and the carboxyterminus. According to this embodiment the samples are first fractionated in one or more low Mr fractions on size using gel filtration chromatography or size exclusion methods (dialysis or ultrafiltration). Thereafter the low Mr protein fractions of the samples are double labelled, without cleavage in between the two labelling steps. In this embodiment of the methods of the invention it is not necessary to use labelled tags, because every protein in the sample is labelled.

In the general methods of the present invention (i.e. where the nature of the functional group is not determined by the nature of the sample or the desired result), the choice of functional groups to be modified on a polypeptide and the order of labelling with isotopic and isobaric labelling reagent can be influenced by different factors. When using commercial labelling reagents, this will, at least in part, be determined by:
- the type of protein reactive groups present on the available labelling reagents
- the presence or absence of an affinity tag in the available labelling reagent.

For specific requirements, thus to perform alternative labelling protocols, labelling reagents other than the present available commercial labelling reagents are prepared.

According to a particular embodiment the double labelling methods of the present invention are performed whereby commercially available labelling reagents are used but wherein novel functional groups are generated on an amino acid. The type of functional groups that can be introduced is mentioned above in the context of protein reactive groups.

The methods of the present invention are useful for the detection of biomarkers, e.g. in the context of disease. Depending on the sample, a large number of peptides are analysed by MS and MS/MS. This amount can be as high as several hundreds or even more than thousand. For many of them, the relative amount in between differentially double-labelled versions of the same peptide will be constant regardless of the origin of the sample. However, significant differences will be observed for a limited number of peptides which are correlated with a condition of the disease as represented by a certain sample. Analysis of these peptides themselves makes it possible to determine the protein from which the peptide originates. The analysis will be even more reliable when it is observed that, for different peptides from a same protein, an identical expression level is detected.

It will be appreciated that the labelling methods of the present invention are applicable in proteomics, protein expression profiling, biomarker discovery and target discovery. The high multiplexing of the methods of the present invention allows to analyse a number of different conditions in one single experiment. The method is particularly useful for studying the expression profile of a sample obtained from different disease states. Samples which are analysed are derived from e.g. a healthy person and one or more conditions representing e.g. different stages of a disease, such as from a person with a benign disorder, from a person with a malignant disorder (mild or agressive). Additionally or alternatively samples for simultaneous analysis can be obtained from persons responding or not responding to a treatment, from different parts of the body to determine whether the disorder which manifests its self in the different parts of the body, from persons before during and after treatment, from persons receiving a different method of treatment and from persons having one or more symptoms of the disorder, but as a result of a different condition. Disorders which are considered in the context of the present invention include, but are not limited to bacterial or viral infections, immunological disorders, cardiovascular disorders and cancer.

In what follows, the different labelling reagents used in the methods of the present invention are described in more detail.

The nature of the isotopic labels used in the context of the present invention is not critical. Typically, isotopic labelling reagents comprise a protein/peptide reactive group (PRG), allowing the covalent binding to a protein or peptide, connected with an isotopic group (IG) which is optionally attached to an affinity tag (A). The presence of an affinity tag is not critical for the use of the isotopic labels in the context of the present invention, as will be clear from the present description. Nevertheless, commercially available isotopic labels typically comprise an affinity tag. Thus isotopic labelling reagents can be represented by the general structure A-IG-PRG or IG-PRG, depending on whether or not an affinity tag is present. Non-limiting examples of labels of this type are disclosed in US6,852,544 and Gygi et al. cited above. Isotopic labels are commercially available e.g. from Applied Biosystems.

According to one embodiment, the detailed structure of the isotopic labelling reagent corresponds to the general formula A-B¹-X¹-(CH₂)ₙ-[X₂-(CH₂)ₘ]ₓ-X₃-(CH₂)ₚ-X⁴-B²-PRG when an affinity tag is present or X¹-(CH₂)ₙ-[X₂-(CH₂)ₘ]ₓ-X₃-(CH₂)ₚ-X⁴-B² -PRG when no affinity tag is present. Wherein:
- X¹-(CH₂)ₙ-[X₂-(CH₂)ₘ]ₓ-X₃-(CH₂)ₚ-X⁴ corresponds to the isotopic group as defined above
- A and PRG are as defined above,
- B¹ and B², independently of one another, are optional moieties that facilitate binding of the A or PRG group to the isotopic group or prevent undesired cleavage of those groups from the isotopic group and are selected, for example, from COO, CO, CO-NR', CS-NR' and optionally contains one or more CH₂ groups alone or in combination with other groups, e.g. (CH₂)_{q}-CONR', (CH₂)_{q}-CS-NR', or (CH₂)_{q},
- X¹, X², X³ and X⁴, independently of one another, are selected from O, S, NH, NR, NRR'⁺, CO, COO, COS, S-S, SO, SO₂, CO-NR', CS-NR', Si-O, aryl or diaryl groups. (R is an alkyl, alkenyl, alkynyl, alkoxy or aryl group and R' is a hydrogen, an alkyl, alkenyl, alkynyl, alkoxy or aryl group). In particular embodiments X¹ -X⁴ are absent, but typically at least one of X¹ -X⁴ is present.
- n, m, p and q are whole numbers with values from 0 to about 100. In particular embodiments one of n, m, p or q is not 0 and x is also a whole number ranging from 0 to about 100 where the sum of n+xm+p+q is less than about 100 and in more particular embodiments less than about 20.

In particular embodiments of the isotopic labels useful in the context of the present invention, one or more of the CH₂ groups of the isotopic group is optionally substituted with small (C₁-C₆) alkyl, alkenyl, or alkoxy groups, an aryl group or is substituted with functional groups that promote ionization, such as acidic or basic groups or groups carrying permanent positive or negative charge. In particular embodiments, one or more single bonds connecting CH₂ groups in the isotopic group are replaced with a double or a triple bond. In particular embodiments R and R' alkyl, alkenyl, alkynyl or alkoxy groups are small, i.e. having 1 to about 6 carbon atoms.

Affinity tags, when present on the isotopic labels used in the context of the present invention, allow the selective binding of peptides or polypeptides comprising the isotopic label, either covalently or non-covalently and with high affinity to a capture reagent (CR). Typically the binding of the affinity tag to the capture reagent is strong such that it is resistant to extensive and/or multiple washing with any or a combination of a variety of solutions which ensure the removal of peptides or polypeptides non-specifically bound to the capture reagent. Typically the affinity tag does not undergo peptide-like fragmentation during MS analysis.

The removal of peptides or polypeptides bound to the CR through an affinity tag can be ensured by addition of a displacing ligand (DL) described below or by changing the temperature or solvent conditions.

As indicated above, the nature of the affinity tag is not critical to the principle of the present invention, as the affinity purification of the labelled peptides or proteins is not critical in the methods and tools of the present invention.

Nevertheless, according to a particular embodiment, it is envisaged that, in order to further reduce the complexity of the sample for analysis, an extraction of the isotopically labelled proteins or peptides is envisaged in the context of the methods and tools of the present invention. For use in the context of these embodiments, the nature of the affinity tags is also not critical, as long as it allows the selective binding to a capture reagent (CR) and optionally removal therefrom, without affecting the peptide or polypeptide bound to the affinity tag.

Accordingly, non-limiting examples of A and CR pairs include:
- d-biotin or structurally modified biotin-based reagents, including d-iminobiotin, which bind to avidin/streptavidin (for example as strepavidin-Agarose, oligomeric-avidin-Agarose, or monomeric-avidin-Agarose)
- a 1,2-diol, such as 1,2-dihydroxyethane (HO-CH₂ -CH₂ -OH), and other 1,2-dihydroxyalkanes including those of cyclic alkanes, e.g., 1,2-dihydroxycyclohexane which bind to an alkyl or aryl boronic acid or boronic acid esters, such as phenyl-B(OH)₂ or hexyl-B(OEthyl)₂ (eg attached via an alkyl or aryl group to a support such as agarose)
- maltose which binds to Maltose Binding Protein; or other sugar/Sugar Binding Protein pairs, or more generally to any ligand/Ligand Binding Protein pair which obeys to the above mentioned criteria of affinity tags.
- a hapten, such as dinitrophenyl group, which binds to the corresponding anti-hapten antibody such as anti-dinitrophenyl-IgG;
- a ligand which binds to a transition metal, for example, an oligomeric histidine (so called 6His-tag) will bind to Ni(II), the transition metal CR is in particular embodiments used in the form of a resin-bound chelated transition metal, such as nitrilotriacetic acid-chelated Ni(II) or iminodiacetic acid-chelated Ni(II);
- glutathione which binds to glutathione-S-transferase.

In particular embodiments of the present invention, an affinity purification of labelled peptides or polypeptides is performed prior to analysis by MS. Accordingly, as the bound peptides or polypeptides are required for further analysis, removal from the capture reagent is required. This can be ensured in different ways.

Where the isotopic labels used in the context of the present invention comprise an affinity tag, this affinity tag can be connected to the labelling reagent by a cleavable bond (such as, but not limited to an acid labile, thiol labile, base labile, periodate labile, or hydroxylamine labile bond).

The bond between affinity tag and isotopic group can also be cleavable, for example, by chemical, thermal or photochemical reaction. A suitbale photocleavable groups is 1-(2-nitrophenyl)-ethyl. Thermally labile bonds are for example, double-stranded nucleic acids, double strands of a nucleic acid with peptide nucleic acid, or double stranded peptide nucleic acid strands which will dissociate upon heating. Cleavable isotopic groups also include those having disulfide bonds, acid or base labile groups, including among others, diarylmethyl or trimethylarylmethyl groups, silyl ethers, carbamates, oxyesters, thiesters, thionoesters, and alpha-fluorinated amides and esters. Enzymatically cleavable isotopic groups are for example, protease-sensitive amides or esters, beta-lactamase-sensitive betalactam analogues and bonds that are nuclease-cleavable, or glycosidase-cleavable.

According to this embodiment, the affinity tag can be removed by treatment of the labelled protein or peptide with a suitable reagent. Removal of the affinity tag may also be desirable for other reasons, e.g. to avoid interference in analysis of the peptide, e.g. in MS. Typically, the affinity tag is cleaved off after the affinity isolation of labelled peptides or polypeptides.

In particular embodiments, biotin and biotin-based affinity tags are used. Of particular interest are structurally modified biotins, such as d-iminobiotin, which will elute from avidin or strepavidin columns under solvent conditions compatible with ESI-MS analysis, such as dilute acids containing 10-20% organic solvent. It is expected that d-iminobiotin tagged compounds will elute in solvents below pH 4.

Additionally or alternatively, displacement ligands (DL) are used to displace the affinity tag A (and the peptide or protein bound thereto) from the CR. When eluting with a DL at least a fraction of this DL will be present in the eluent comprising the peptide(s) of interest. In particular embodiments *the methods of the invention can comprise the use of a DL which is a molecule which does not undergo peptide-like fragmentation during MS analysis, and of which the presence in a sample does not significantly suppress the ionization of the tagged peptide, substrate or reaction product conjugates. Particularly, the displacement ligand can be chosen such that it itself is minimally ionized during mass spectrometric analysis and that the formation of ions composed of DL clusters is minimal.

The nature of a suitable displacement ligand (DL), depends upon the A and CR that are employed. In general, the DL is selected to displace A from CR in a reasonable time scale, at most within a week of its addition, but more preferably within a few minutes or up to an hour. The affinity of DL for CR should be comparable or stronger than the affinity of the tagged compounds containing A for CR. Furthermore, the DL should be soluble in the solvent used during the elution of tagged compounds containing the affinity tag A, from CR. In particular embodiments the DL corresponds to a free affinity tag A or a derivative or structural modification of A. Examples of displacement ligands thus include, d-biotin or d-biotin derivatives.

The isotopic groups, present in the isotopic labels used in the context of the present invention comprise a structure wherein one or more of the atoms in the isotopic group are substituted with a stable isotope, so as to generate one or more compounds with the same chemical formula, but which are isotopically distinguishable based on their difference in mass. For example, any one or more of the hydrogen, nitrogen, oxygen or sulphur atoms in the isotopic group can be replaced with their isotopically stable isotopes ²H, ¹³C, ¹⁵N, ¹⁷O, ¹⁸O or ³⁴S, respectively. For example, hydrogen is substituted with deuterium or carbon ¹²C is substituted with ¹³C. Using one more of the above isotopes, 2, 3, 4, 5, 6, 7, 8, or even more isotopic labels can be produced each having the same structure but with a different Mr, which is reflected in the Mr of the polypeptide or peptide to which it has been bound.

Examples of Isotopic groups include among others: ethers, polyethers, ether diamines, polyether diamines, diamines, amides, polyamides, polythioethers, disulfides, silyl ethers, alkyl or alkenyl chains (straight chain, branched or with portions which are cyclic), aryl, diaryl or alkyl-aryl groups. In particular embodiments, aryl groups present in the isotopic groups of the isotopic labels contain one or more heteroatoms (e.g., N, O or S atoms).

According to a particular embodiment, the isotopic label is such that it does not undergo peptide-like fragmentation during (MS)ⁿ analysis. To promote ionization, the isotopic label, and more particularly the isotopic group therein, may contain groups or moieties such as acidic or basic groups, e.g., COOH, SO₃H, primary, secondary or tertiary amino groups, nitrogen-heterocycles, ethers, or combinations of these groups. In particular embodiments, the isotopic group contains groups having a permanent charge, e.g., phosphonium groups, quaternary ammonium groups, sulfonium groups, chelated metal ions, tetralky or tetraryl borate or stable carbanions.

The protein/peptide reactive group (PRG) on the labelling reagent is a group that selectively reacts with certain protein or peptide functional groups or is a substrate of an enzyme of interest. Examples of suitable protein/peptide reactive groups are:
- Thiol-reactive groups react with the side chain of cysteine. Thiol-reactive groups include epoxides, alpha-haloacyl group, nitriles, sulfonated alkyl or aryl thiols, alkyl halides, aryl amides and maleimides. A particular example is iodoacetamide or a derivative thereof.
- Amino-reactive groups react with the epsilon amine group of the side chain of lysine or react with the amine at the aminoterminus of a polypeptide. Amino reactive groups tag amino groups in proteins and include sulfonyl halides, isocyanates, isothiocyanantes, active esters, including tetrafluorophenyl esters, pentafluorophenyl esters, N-hydroxysuccinimidyl esters, N-hydroxysulfosuccinimidyl esters, 2-nitrophenyl esters, 4-nitrophenyl esters, 2,4-dinitrophenylesters and 2,4-dihalophenyl esters, acid halides, and acid anyhydrides and mixed anhydrides. In addition, amino reactive groups include aldehydes or ketones in the presence or absence of NaBH₄ or NaCNBH₃.
- Carboxylic acid-reactive groups react with the side chains of aspartic aicd and glutamic acid or react with the carboxyterminus of a polypeptide. Carboxylic acid reactive groups include amines or alcohols in the presence of a coupling agent such as dicyclohexylcarbodiimide, or 2,3,5,6-tetrafluorophenyl trifluoroacetate and in the presence or absence of a coupling catalyst such as 4-dimethylaminopyridine; and transition metal-diamine complexes including Cu(II)phenanthroline.
- Imidazoles reactive groups react with histidine
- Ester reactive groups include amines which, for example, react with homoserine lactone. Methionine is converted upon homoserine lactone during CNBr cleavage.
- Phosphate reactive groups react with phosphorylated amino acids such as phosphoSer, PhosphoThr and PhosphoTyr. Phosphate reactive groups include chelated metal where the metal is, for example Fe(III) or Ga(III), chelated to, for example, nitrilotriacetiac acid or iminodiacetic acid. These agents react with posphorylated amino acids (such as phosphoserine, phosphothreonine, and phosphotyrosine).
- Aldehyde or ketone reactive groups include amine plus NaBH4 or NaCNBH3, or these reagents after first treating a carbohydrate with periodate to generate an aldehyde or ketone.
- Hydroxyl reactive groups react with serine and threonine. Hydroxyl reactive groups include trityl-halides or a silyl-halide reactive moiety which is either substituted or unsubstituted.

The isotopic labels of the present invention are bound to a protein or peptide by interaction between the protein/peptide reactive group of the labelling reagent and a functional group present within the protein or peptide. This functional group can be pre-existing in the polypeptide as present in the sample or can be created thereon, optionally after the isolation of the polypeptide. For example, a carboxylic acid group can be modified with water-soluble carbodiimide (e.g. 1-(3-dimethylaminopropyl)-3-ethylcarbodiimide hydrochloride; EDC) to thereby obtain an electrophilic group that reacts with a nucleophile such as an amine group. Activation of the carboxylic acid group of a labeling reagent with EDC can be performed in the presence of an amine. The use of carbodiimides in combination with NH₂-CH₂-CH₂-NH₂ allows the conversion of carboxylgroups into amine groups. The use of EDC in combination with NH₂-CH₂-CH₂-SH together ensures the conversion of carboxylgroups into thiol groups.

A non-limiting list of other compounds which are suitable to modify or to introduce functional groups on a peptide or polypeptide includes 2-Aminoethyl-2'-amino-ethanethiolsulfonate which is a thiol-reactive amination reagent that converts a thiol group into a reducing agent cleavable amine group; aminoethyl-8 reagent that converts free thiols into primary amine groups; BMPA that modifies thiol groups to carboxyl groups; MSA which is amine-reactive with latent carboxyl group (it converts amines to protected carboxyls. Carboxyls are unmasked at pH 9.5 in phosphate buffer); SATA which reacts with primary amines to add protected thiols; Hydroxylamine HCl which is a highly effective reagent for deblocking SATA and SATP modified proteins to generate a free thiol and Traut's Reagent which is a water-soluble reagent that reacts with primary amines at pH 7-10 to introduce thiol groups. Such reagents available from e.g. Pierce Chemicals (IL, USA).

It is also envisaged that one or more functional groups are created in a peptide or polypeptide by removal of a protecting group. Consequently, in particular embodiments, functional groups in a polypeptide are changed or added. In particular embodiments the added functional groups are groups which do not occur naturally in proteins.In other embodiments of the isotopic labelling reagents suitable in the context of the present invention, the PRG groups are in fact substrates for a particular protein-modifying enzyme. This enzyme can be an enzyme which is involved, in vivo, in post translation modifications. Some of these enzymes are associated with a disease state or birth defect. Examples hereof are acid phosphatase, alkaline phosphatase, alanine aminotransferase, amylase, angiotensin converting enzyme, aspartate aminotransferase, creatine kinase, gamma-glutamyltransferase, lipase, lactate dehydrogenase, and glucose-6-phosphate dehydrogenase, proteases, esterases.

In particular embodiments the isotopic labelling reagent is a compound with general structure: 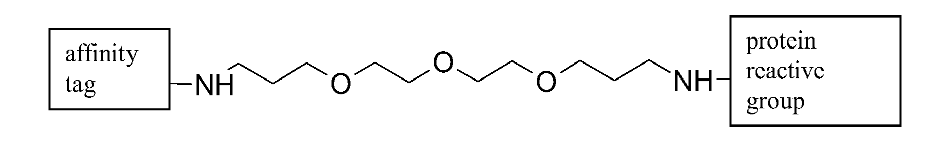 wherein one or more atoms in the linker between the affinity tag and the protein reactive group is ¹³C and/or deuterium. In even more particular embodiments, the affinity tag is a biotin label and the protein/peptide reactive group is reactive towards the cysteine amino acid.

In a further particular embodiment, the isotopic labelling reagent is a compound with structure: 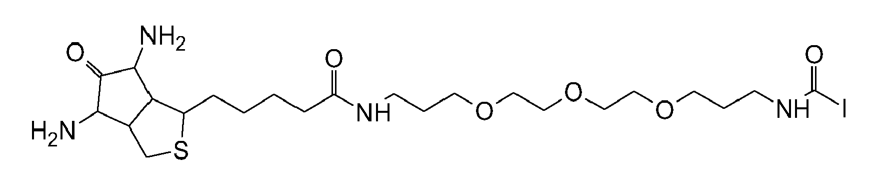 wherein one or more atoms in the linker between the biotin and iodoacetamide group are 13C and/or deuterium.

As detailed above, the methods of the present invention relate to the combined use of two different types of labels, to increase multiplexing capacity of isotope-based labels. More particularly, according to the present invention, the use of isotopic labels is combined with isobaric labels. Isobaric labelling reagents are described i.a. in WO2004070352 and Ross et al. cited above and are commercially available from Applied Biosystems.

As is the case for isotopic labelling reagents, isobaric labelling reagents optionally comprise an affinity tag, depending on their use in the double labelling methods of the present invention.

According to one embodiment, the labelling reagents for attaching an isobaric label without affinity tag can be represented by the general formula RP-X'-BG-Y'-PRG wherein RP is a reporter group, X' and Y' both represent a bond or link, BG is a balance group between links X' and Y', and PRG is a protein/peptide reactive group, that specifically reacts with a functional group of a protein or peptide. Accordingly, the labelled protein/peptide is represented by the general formula: RP-X'-BG-Y'-protein/peptide.

The nature of the protein reactive group present in an isobaric labelling reagent is the same as described above for isotopic labels. The choice of a particular PRG depends on the order of labelling and the type of functional groups that are to be labelled. In a particular embodiment, the PRG in an isobaric labelling reagent is reactive against primary amines.

The concept of isobaric labelling is exemplified in Figure 3. In this embodiment, the complete isobaric labelling reagent consists of a reporter group based on N-methylpiperazine, a mass balance group which is a carbonyl, and a peptide-reactive group which is an NHS ester. While the mass of the reporter group is specific for each isobaric label within a set, the overall mass of the reporter group and the balance group of the different isobaric labels are kept constant. According to a particular embodiment this is ensured by using differential isotopic enrichment with ¹³C, ¹⁵N, and ¹⁸O atoms as indicated, to avoid problems with chromatographic separation seen with enrichment involving deuterium substitution. In view of the identical structure of the different isobaric reagents, the number and position of enriched centers in the ring has no effect on chromatographic or MS behaviour.

Upon reaction of the amine specific reactive group of this labelling reagent with a peptide, the label becomes connected via an amide linkage to amine functional groups (N-terminal or amine group of lysine). These amide linkages fragment in a similar fashion to backbone peptide bonds when subjected to CID. Other suitable methods to fragment peptides include CAD (collisionally activated dissociation), ETD (electron transfer dissociation), ECD (electron capture dissociation), IRMPD (infrared multiphoton dissociation) and BIRD (blackbody infrared radiative dissociation. Following fragmentation of the amide bond, however, the balance (carbonyl) moiety is lost (neutral loss), while charge is retained by the reporter group fragment. The numbers in parentheses indicate the number of enriched centres in each section of the molecule. Part B of Figure 3 illustrates the isotopic tagging used to arrive at four isobaric combinations with four different reporter group masses. A mixture of four identical peptides each labelled with one member of the multiplex set appears as a single, unresolved precursor ion in MS (identical m/z). Following CID, the four reporter group ions appear as distinct masses (114-117 Da). All other sequence-informative fragment ions (b-, y-, etc.) remain isobaric, and their individual ion current signals (signal intensities) are additive. This remains the case even for those tryptic peptides that are labelled at both the N terminus and lysine side chains, and those peptides containing internal lysine residues due to incomplete cleavage with trypsin. The relative concentration of the peptides is thus deduced from the relative intensities of the corresponding reporter ions. In contrast to ICAT and similar mass-difference labelling strategies, quantitation is thus performed at the MS/MS stage rather than in MS.

As indicated above, the reporter group RP of an isobaric labelling reagent is a group that has a unique mass (or mass to charge ratio) that can be determined. Accordingly, each reporter of a set of isobaric labels has a unique mass. In particular embodiments, each reporter group of a set of isobaric labels comprises one or more heavy atom isotopes to achieve the unique mass. For example, isotopes of carbon (¹²C, ¹³C and ¹⁴C), nitrogen (¹⁴N and ¹⁵N), oxygen (¹⁶O and ¹⁸O) or hydrogen (hydrogen, deuterium and tritium) can be used in the preparation of differing reporter groups. Examples of stable "heavy" atom isotopes suitable for use in the reporter group include ¹³C, ¹⁵N, ¹⁸O and deuterium. These are not limiting as other light and heavy atom isotopes are also suitable for incorporation in the reporter group. Basic starting materials suitable for preparing reporter groups comprising light and heavy atom isotopes are available from various commercial sources such as Cambridge Isotope Laboratories and Isotec. According to one embodiment, the reporter group ranges in mass from m/z 114.1 to 117.1, while the balance group ranges in mass from 28 to 31 Da, such that the combined mass remains constant (145.1 Da) for each of the four reagents.

According to the present invention, the unique reporter group is used to identify and quantitate a peptide in a sample. In a particular embodiment, the unique reporter group is present as part of a label on one or multiple polypeptides of a sample. In this way information about the reporter group is associated with information about labelled polypeptides of the sample.

Alternatively, however, the reporter group is removed from the labelled polypeptide prior to its identification. Thus, the reporter group need not be physically linked to a (poly)peptide at the moment when the reporter is determined. Indeed, according to this embodiment, the unique mass of the reporter is, for example, determined in a second mass analysis of a tandem mass analyzer, after ions of a labelled (poly)peptide are fragmented to produce daughter fragment ions of the (poly)peptide and detectable reporter groups. The particular reporter group is used to identify the sample from which a particular (poly)peptide originated. Further, the determined quantity of the unique reporter group, either relative to the amount of other reporter groups or relative to a calibration standard (e.g. a polypeptide labelled with a specific reporter group), is optionally used to determine the relative or absolute amount (often expressed as a concentration and/or quantity) of polypeptide in the sample or samples. Therefore the reporter group that is used to label each particular sample provides different types of information, such as the amount of one or more polypeptides in a particular sample. Where the identity of a polypeptide is also determined, that information is correlated with information pertaining to the different reporter groups to thereby facilitate the determination of the identity and amount of each labelled polypeptide in one or a plurality of samples.

The reporter either comprises a fixed charge or is capable of becoming ionised. Accordingly, the isobaric labelling reagent is used to label the reactive polypeptide in a salt or zwitterionic form. Ionisation of the reporter facilitates its determination in a mass spectrometer. Accordingly, in particular embodiments the reporter is determined as a ion, sometimes referred to as a "signature ion". When ionised, the reporter comprises one or more net positive or negative charges. For example, the reporter group comprises one or more basic nitrogen atoms (positive charge) or one or more ionizable acidic groups such as a carboxylic acid group, sulfonic acid group or phosphoric acid group (negative charge). Non-limiting examples of reporter groups comprising a basic nitrogen include substituted or unsubstituted morpholines, piperidines or piperazines.

In particular embodiments, the reporter is a 5, 6 or 7 membered heterocyclic ring comprising a ring nitrogen atom that is N-alkylated with a substituted or unsubstituted acetic acid moiety to which the polypeptide is linked through the carbonyl carbon of the N-alkyl acetic acid moiety, wherein each different isobaric label comprises one or more heavy atom isotopes. This heterocyclic ring is substituted or unsubstituted. The heterocyclic ring is aliphatic or aromatic. Possible substituents of the heterocyclic moiety include alkyl, alkoxy and aryl groups. The substituents comprise protected or unprotected groups, such as amine, hydroxyl or thiol groups, suitable for linking the polypeptide to a support. In particular embodiments, the heterocyclic ring comprises additional heteroatoms such as one or more nitrogen, oxygen or sulfur atoms. In particular embodiments, the reporter is selected such that it does not substantially sub-fragment under conditions typical for the analysis of the polypeptide such as electrophoresis or chromatography. In other particular embodiments, the reporter is chosen so that it does not substantially sub-fragment under conditions of dissociative energy applied to cause fragmentation of both bonds X' and Y' of at least a portion of selected ions of a labelled polypeptide in a mass spectrometer. Optionally, the reporter group is chosen such that eventual fragments of the reporter group are difficult or impossible to detect above background noise using MS analysis. In particular embodiments, the mass of a reporter group is intentionally selected to be different compared with the mass of the polypeptide sought to be determined or any of the expected fragments of the polypeptide. For example, the reporter's mass is chosen to be different compared to any naturally occurring amino acid or peptide, or expected fragments thereof. This facilitates polypeptide determination since, depending on the polypeptide, the lack of any possible components of the sample having the same coincident mass adds confidence to the result of any analysis. In particular embodiments, the reporter is a small molecule that is non-polymeric. In further particular embodiments, the mass of a reporter is less than 250 Daltons. Such a small molecule is easily determined in the second mass analysis, free from other components of the sample having the same coincident mass in the first mass analysis. In this context, the second mass analysis is performed typically in a tandem mass spectrometer, on selected ions that are determined in the first mass analysis. Because ions of a particular mass to charge ratio are specifically selected out of the first mass analysis for possible fragmentation and further mass analysis, the non-selected ions from the first mass analysis are not carried forward to the second mass analysis and therefore do not contaminate the spectrum of the second mass analysis. Furthermore, the sensitivity of a mass spectrometer and the linearity of the detector (for purposes of quantitation) is quite robust in this low mass range. Additionally, the present state of mass spectrometer technology allows for baseline mass resolution of less than one Dalton in this mass range.

The balance group (BG) of the isobaric labelling reagent links the reporter to the protein reactive group. In particular embodiments the balance group is selected to produce a neutral species when both bonds X' and Y' are fragmented (i.e. undergoes neutral loss upon fragmentation of both bonds X' and Y'). In particular embodiments, the balance group is a very small moiety such as a carbonyl or thiocarbonyl group. For example, the balance group comprises at least one heavy atom isotope and comprises the formula -CH₂-CO-CH₂-, CH₂-CS-CH₂-, CH₂-CNH-CH₂- or CH₂-CHR¹-CH₂-, wherein R¹ is the same or different and is an alkyl group comprising one to eight carbon atoms which optionally contain a heteroatom or a substituted or unsubstituted aryl group wherein the carbon atoms of the alkyl and aryl groups independently comprise linked hydrogen, deuterium and/or fluorine atoms. In particular embodiments, the balance group is a larger moiety. For example, the balance group is a polymer or a biopolymer. In particular embodiments, the balance group is designed to sub-fragment when subjected to dissociative energy levels, including sub-fragmentation to thereby produce only neutral fragments of the balance group. Typically, the balance group of the isobaric labels comprises one or more heavy atom isotopes such that its mass compensates for the difference in mass between the reporters groups, so as to obtain an identical mass for each isobaric label of the set. The aggregate mass (i.e. the mass taken as a whole) of the reporter/balance group combination is the same for each label and thus the mass of identical polypeptides or peptides, originating from different samples and labelled with different isobaric labels of a set will be the same. Identical peptides or polypeptides labelled with different isobaric labelling reagents (of the same set) are isobars, i.e. have the same weight. Thus, if one selects, from an initial mass analysis of a pooled sample mixture, an ion of a particular mass to charge ratio in the mass spectrometer, this selected ion contains all identical polypeptides from the different samples that make up the pooled sample mixture, in a proportion which corresponds to their respective concentration and/or quantity in the sample mixture. Because the balance group acts as a mass balance for the reporter in the isobaric labelling reagents, such that the aggregate mass of the reporter group/balance group combination is the same for all labelling reagents of a set or kit, the greater the number of atoms in the balance group, the greater the possible number of different isomeric/isobaric labelling reagents within a set and/or kit. Stated differently, generally the reporter group is not a limiting factor and the greater the number of atoms that a balance group comprises, the greater number of potential reporter/balance group combinations exist, since isotopes can be substituted at most any position in the balance group to thereby produce isomers or isobars of the balance group portion wherein the balance group portion is used to offset the differing masses of the reporter portion and thereby create a set of reporter/balance group isomers or isobars. Such diverse sets of isobaric labelling reagents are particularly well suited for multiplex analysis of polypeptides in the same and/or different samples.

The total number of different isobaric labelling reagents in a multiplex experiment is two, three, four, five, six, seven, eight, nine, ten or more. The diversity of isobaric labelling reagents is limited only by the number of atoms of the reporter and balance group moieties, the heavy atom isotopes available to substitute for the light isotopes and the various synthetic configurations in which the isotopes can be synthesized. As mentioned, numerous isotopically enriched basic starting materials are readily available from manufacturers such as Cambridge Isotope Laboratories and Isotec. Such isotopically enriched basic starting materials are used in the synthetic processes used to produce sets of isobaric and isomeric labelling reagents or to produce the isotopically enriched starting materials that are used in the synthetic processes used to produce sets of isobaric and isomeric labelling reagents. Examples of the preparation of isobaric labelling reagents suitable for use in a multiplexing labelling experiment are disclosed in the examples of W02004070352.

In the isobaric labels used in the context of the present invention, the reporter group, balance group and protein/peptide reactive group, are connected by bonds referred to as X' and Y', wherein X' is a bond between an atom of the reporter group and an atom of the balance group and Y' is a bond between an atom of the balance group and an atom of the protein/peptide reactive group. In particular embodiments bonds X' and Y' of the various isobaric labelling reagents (i.e. RP-X'-BG-Y'-PRG) are dissociated, when subjected to dissociative energy levels. Therefore, in particular embodiments of the methods of the invention, the dissociative energy level is adjusted in a mass spectrometer so that both bonds X' and Y' are dissociated in at least a portion of the selected ions of the isobarically labelled polypeptides or peptides (i.e. RP-X'-BG-Y'-Polypeptide/peptide). Fragmentation of bond X' releases the reporter group from the polypeptide so that the reporter is detectable independently from the peptide or polypeptide. Fragmentation of bond Y' releases the reporter group/balance group combination from the polypeptide or peptide, or the balance group from the polypeptide, depending on whether or not bond X' has already been fragmented. in particular embodiments bond Y' is more labile than bond X'. In other particular embodiments bond X' can be more labile than bond Y'. In yet other particular embodiments, bonds X' and Y' have the same relative lability. In particular embodiments, the relative lability of bonds X' and Y' is adjusted with regard to an amide (peptide) bond. Bond X', bond Y' or both bonds X' and Y' are in such case more, equally labile or less labile compared to a typical amide (peptide) bond. For example, under conditions of dissociative energy, bond X' and/or bond Y' are less prone to fragmentation as compared with the peptide bond of a Z-Pro dimer or Z-Asp dimer, wherein Z is any natural amino acid and Pro and Asp are respectively proline and aspartic acid. In some embodiments, bonds X' and Y' will fragment with approximately the same level of dissociative energy as a typical amide bond. In some embodiments, bonds X' and Y' will fragment with a greater level of dissociative energy as compared with a typical amide bond.

In other particular embodiments, bonds X' and Y' are chosen such that fragmentation of bond Y' induces the fragmentation of bond X', and vice versa. In this way, both bonds X' and Y' fragment essentially simultaneously such that no substantial amount of polypeptide or peptide, or daughter fragment ion thereof, comprises a partial label in the second mass analysis. Substantial amount of polypeptide refers to less than 25 %, and even less than 10%, partially labelled polypeptide that is determined in the MS/MS spectrum. Because there can be a clear demarcation between labelled and unlabelled fragments of the polypeptide in the spectra of the second mass analysis (MS/MS), this feature simplifies the identification of the polypeptides based on computer assisted analysis of the daughter fragment ion spectra. Moreover, because the fragment ions of polypeptides are, in some embodiments, either fully labelled or unlabelled (but not partially labelled) with the reporter/balance group moiety, there is little or no scatter in the masses of the daughter fragment ions caused by isotopic distribution across fractured bonds such as would be the case where isotopes were present on each side of a single labile bond of a partially labelled polypeptide routinely determined in the second mass analysis.

Particular embodiments of isobaric labelling agents are disclosed in detail from page 26 to 50 of WO 2004070352. Other particular embodiments of isobaric labelling agents are disclosed in Figure 3 herein (Taken from Ross et al. cited above). The agent consists of a reporter group (based on N-methylpiperazine), a mass balance group (carbonyl), and a peptide-reactive group (NHS ester). The overall mass of reporter and balance components of the molecule are kept constant using differential isotopic enrichment with ¹³C, ¹⁵N, and ¹⁸O atoms as indicated in the figure The reporter group ranges in mass from m/z 114.1 to 117.1, while the balance group ranges in mass from 28 to 31 Da, such that the combined mass remains constant (145.1 Da) for each of the four reagents.

As indicated above, isobaric labelling reagents can further comprise an affinity tag A. In a embodiment the affinity tag present in the isobaric label is such as described for the affinity tag in the section of isotopic labels. In principal the affinity tag is located at any of RP, X' Y' or BG, but in order to interfere as little as possible with the balance group/reporter group system, as explained below, the affinity group is positioned on X' or Y' or is provided as an additional moiety between Y' and PRG. Generally the affinity tags are cleavable tags which are removed from the label prior to detection, leaving only a scar on the X' or Y' bond if placed there.

In particular embodiments the isobaric label reagent with an affinity tag has a general structure RP-X'-BG-Y'-A-PRG wherein the affinity tag is located as a side group on an interconnecting part between Y' and PRG, such that the tag is cleavable from the isobaric reagent without breaking the bond between PRG and the balance group-reporter combination.

In methods which make use of a tagged isobaric reagent it is possible to label a protein at a functional group, cleave the labelled protein into peptides and specifically isolate the labelled peptide with the affinity tag which is present on the label. This in contrast with the commonly used isobaric labelling methods wherein labelled peptides can not be purified/isolated from unlabelled peptides, which requires that every peptide from a protein sample is examined for the presence or absence of a label

Other arrangements of the systems and methods embodying the invention will be obvious for those skilled in the art.

It is to be understood that although preferred embodiments, specific constructions and configurations, as well as materials, have been discussed herein for devices according to the present invention, various changes or modifications in form and detail may be made without departing from the scope and spirit of this invention.

Yet another aspect of the invention relates to a method wherein isotopic and isobaric labelling is combined and which is analogous to the methods described, with the exception that the isotopic labelling is not performed by covalently attaching an isotopic labelling reagent via a protein reactive group as disclosed above, but by enzymatically cleaving the samples in the presence or absence of H₂¹⁸O.

According to a particular embodiment, the step of enzymatic cleavage is performed by treatment of the samples with trypsin, in the presence of either normal water (H₂¹⁶O) or isotopically labelled H₂¹⁸O. Details on the trypsin mediated ¹⁸O incorporation are for example given in Heller et al. (2003) J. Am. Soc. Mass Spectrom. 14(7), 704-718. Upon enzymatic cleavage with trypsin, two O atoms are incorporated in the C-terminus of newly generated peptides. Accordingly, when this reaction is performed in the presence of H₂¹⁸O, two ¹⁸O atoms are incorporated in the C-terminus of newly generated peptides. A C-terminal peptide of a protein which does not comprise a C-terminal Lysine or an Arginine will not have an ¹⁸O atom incorporated.

According to one embodiment, the double labelling methods of the present invention involve the differential isotopic labelling of half of the samples by introduction of either two ¹⁸O or two ¹⁶O by the combined cleavage and labelling step with trypsin described above. The resulting peptides become isotopically labelled with a mass difference of 4, without the attachment of additional groups to the peptide.

According to alternative embodiments, enzymes other than trypsin are used such as Lys-C, or Glu-C.

According to yet another embodiment, the step of cleaving of the proteins is performed using Peptidyl-Lys metalloendopeptidase (Lys-N). Cleavage with Lys-N results in the incorporation of only one ¹⁸O atom in the resulting peptide, which results in a mass difference of 2 between labelled and unlabelled species. This has the advantage that this enzyme does not generate a mixture of isotopically labelled peptides resulting from the incorporation of one or two ¹⁸O atoms into a peptide. Also Asp-N and chymotrypsin incorporate a single ¹⁸O upon cleavage (Schnolzer et al. (1996) Electrophoresis 17, 945-953).

In the double labelling methods of the present invention involving the use of trypsin mediated labelling, the reduction in sample complexity is performed by using, as a second labelling reagent (independent of the order in which the first and the second labelling reagent is used) an isobaric labelling reagent reactive to a specific functional group and carrying an affinity tag. Alternatively, when the isobaric labelling reagent does not comprise an affinity tag, an additional affinity step is performed, which selects certain peptides from a peptide mixture. For example, Cysteine comprising peptides are reversibly bound to thiol-comprising matrices under oxidising conditions, or Histidine comprising peptides are bound to IMAC affinity matrices.

Isotopic labelling with ¹⁸O has also certain advantages. Labelling with ¹⁸O labelled water is cheaper than with other more complex isotopic labelling reagents. Further, isotopic labelling and protein cleavage are performed in one step, which simplifies the double labelling method and increases the reproducibility. The ¹⁸O labelling also ensures that each cleaved peptide is also effectively labelled with the isotope. Protease mediated labelling at the C-terminus does not change the physicochemical properties of the peptide. This can be of importance for the chromatographic behaviour of peptides on e.g. ion exchange chromatography, isoelectrofocusing and reversed phase chromatography.

Accordingly particular embodiments of the invention relates to double labelling methods wherein in a first step two or more samples are labelled with a set of protein reactive isobaric labelling reagent with an affinity tag. In a next step the labelled proteins are cleaved with an protease (e.g. trypsin) whereby in a part of the samples this cleavage is performed in the presence of H₂¹⁸O. In a next step, the complexity of the samples are reduced by an affinity isolation for the affinity tag on the isobaric labelling reagent.

In an alternative embodiment of the method, the samples are in a first step cleaved with an protease whereby a part of the samples is labelled by protease mediated ¹⁸O incorporation. In a next step the complexity of the samples is reduced by isolating peptides with specific amino acids (e.g. Cysteine and Histidine as described above). In a next step the isolated peptides are labelled with an isobaric labelling reagent. This labelling can be done at the N-terminus of the isolated peptides, or on the functional group of the peptides which has been used for isolation. In this method there is no need for the presence of an affinity tag on the isobaric labelling reagent.

In yet an alternative embodiment of the method, the proteins are cleaved as described in the previous method. In a next step the cleaved proteins are labelled with an isobaric labelling reagent comprising an affinity tag. The protein reactive group on the labelling reagent should not react with the N-terminus as this functional group is present in all cleaved peptides and will not reduce the complexity of the sample. Also the use of carboxyl reactive groups for isobaric labelling needs caution. Depending on the type of reagent an ¹⁸O atom can be removed upon reaction of an isotopic labelled C-terminus with an isobaric labelling reagent. In the case of trypsin mediated isotopic labelling, one of the two incorporated isotopes will still remain, but with other proteases, wherein only one ¹⁸O atom is incorporated, the labelling on carboxyl groups may cause the loss of the isotopic label. In particular embodiments, Cysteine reactive groups are used for isobaric labelling. In a next step, the complexity of the samples is reduced by affinity purification using the affinity tag of the isobaric labelling reagent.

The use of protease mediated ¹⁸O isotopic labelling merely differs from the earlier described isotopic labelling in that a traceless label is incorporated. All the other aspects of the present invention, as described before, such as the pooling of samples, isolation of differentially labelled versions of a peptide and the analysis by MS and interpretation of the results are also applicable to double labelling methods wherein protease mediated ¹⁸O isotopic labelling is performed.

### EXAMPLES

### Example 1: Determination relative expression levels of individual peptides using isotopic and isobaric double labelling.

Using unique combinations of isobaric and isotopic labels it is possible to determine the relative amount of an individual peptide within a mixture of double labelled peptides with different isobaric and isotopic labelling combinations. This is exemplified by the following theoretical example. An internal peptide of 8 samples (1 to 8) is labelled first with isotopic label a or b and thereafter with isobaric labels A, B, C and D according to the scheme in the following Table.

**Table 1.: Determination of relative concentrations of a double labelled polypeptide in a mixture**

| Peptide | 1 | 2 | 3 | 4 | 5 | 6 | 7 | 8 |
|---|---|---|---|---|---|---|---|---|
| Isotopic label | a | a | a | a | b | b | b | b |
| Isobaric label | A | B | C | D | A | B | C | D |
| Pooled sample | 1aA, 2aB, 3aC, 4aD, 5bA, 6bB, 7bC, 8bD | | | | | | | |
| First separation MS | 1aA, 2aB, 3aC, 4aD | | | | 5bA, 6bB, 7bC, 8bD | | | |
| Ratio isotopic label | 2 | | | | 3 | | | |
| Second separation MS/MS | 1aA | 2aB | 3aC | 4aD | 5bA, | 6bB, | 7bC, | 8bD |
| Ratio isobaric label | 1 | 9 | 3 | 7 | 1 | 8 | 6 | 5 |
| Individual ratio | 1/20 x 2/5 = 0.02 | 9/20 x 2/5 = 0.18 | 3/20 x 2/5 = 0.06 | 7/20 x 2/5 = 0.14 | 1/20 x 3/5 = 0.03 | 8/20 x 3/5 = 0.24 | 6/20 x 3/5 = 0.18 | 5/20 x 3/5 = 0.15 |
| Norma-lised | 1 | 9 | 3 | 7 | 1.5 | 12 | 9 | 7.5 |

Thereafter all samples are pooled and subjected to chromatography wherein a double labelled peptides behave in the same manner regardless from the particular isotopic isobaric label combination on a peptide. The different double-labelled versions of the peptide elute as one fraction. The fraction is applied on an MS where it will separate in a fraction with high isotope and a fraction with light isotope. Of these two fractions the relative amount can be calculated. In table 1, 50 % more peptide with the b isotope is measured than with the a isotope. Subsequently, the two peptide fractions with different isotopic labels are fragmented which allows to determine the relative ratio of isobaric peptides within each peptide fraction with a given isotopic label (theoretical ratio's indicated in table 1).

The present example implicitly suggests that equal amounts of sample were used in the method. Inbetween two or more samples of e.g. the same tissue, the majority of proteins are expressed at a same level. Peptide peaks from such proteins are expected to appear at about equal concentrations upon MS analysis. However, striking differences in sample amount (errors in initial concentration, partial loss during the labelling procedure) will be notified during the MS analysis. Namely for nearly each peptide that originates from a sample wherein less material is present, the relative amount of such peptide in a mixture will be nearly always lower compared with the peptides of the other samples.

## Claims

1. A method for simultaneously analysing the presence of one or more polypeptides comprising a first and a second functional group in different samples, which comprises the steps of:
a) a first labelling step wherein a first set of labelling reagents is reacted with a first functional group of the one or more polypeptides in the samples, or peptides generated therefrom,
b) a second labelling step wherein a second set of labelling reagents, is reacted with a second functional group on the polypeptides in the samples, or peptides generated therefrom
wherein one of the first and the second set of labelling reagents is a set of isobaric labelling reagents and the other is a set of isotopic labelling reagents, and wherein the combination is performed such that each of the samples is labelled with a different combination of the first and the second labelling reagents from said first and set second set of labelling reagents.
c) pooling the different samples to obtain a polypeptide sample mix
d) selectively isolating the double-labelled polypeptides from the polypeptide sample mix
e) analysing the relative occurrence of the isolated double-labelled polypeptides by Mass spectroscopy.

2. The method according to claim 1, wherein the first set of labelling reagents are isotopic labelling reagents and the second set of labelling reagents are isobaric labelling reagents.

3. The method according to claim 1, wherein the first set of labelling reagents comprises an affinity tag.

4. The method according to claim 1, wherein after reaction with the first set of labelling reagents, the polypeptides present in the samples are cleaved into peptides.

5. The method according to claim 4, wherein the cleavage is performed with trypsin.

6. The method according to claim 4, wherein the peptides are isolated with the affinity tag on the first set of labelling reagents.

7. The method according to claim 1, wherein the first functional group is a thiol.

8. The method according to claim 1, wherein the second functional group is a primary amine.

9. The method according to claim 1, wherein the isotopic labelling reagent has the formula 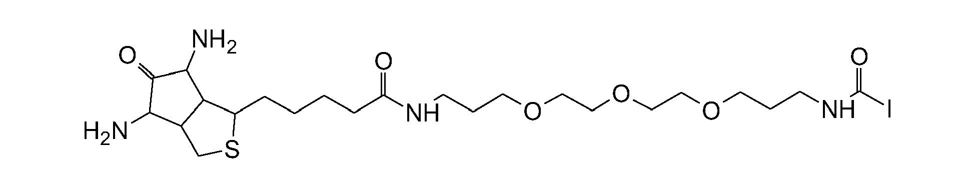 wherein one or more ¹³C isotopes are present in the linker between the biotin and iodoacetamide group.

10. The method according to claim 1, wherein the set of isobaric labelling reagents all comprise a molecule comprising a methyl piperazine derived moiety, a carbonyl moiety and a protein reactive moiety with the formula 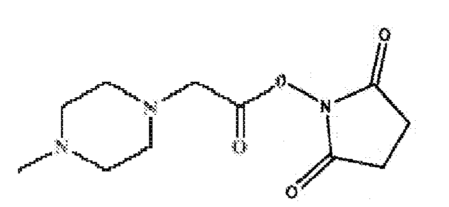 wherein one or more of the atoms in the methyl piperazine derived moiety or in the carbonyl moiety is an isotope selected from the group consisting of ¹⁸O, ²H, ¹⁵N and ¹³C.

11. The method according to claim 1, wherein the first or second functional group of the polypeptides or peptides generated therefrom are present as a result of a modification of another functional group.

12. The method according to claim 1, further comprising the steps of pooling polypeptides or peptides from two or more different samples after the first labelling step.

13. The method of claim 1, wherein step (d) comprises the step of isolating a double-labelled polypeptide or peptide fraction from said pooled polypeptides by electrophoresis or chromatography.

14. The method of claim 1, wherein step (e) comprises the step of determining with MS the relative amount of different isotopes in said isolated double labelled polypeptide or peptide fraction.

15. The method of claim 14, wherein step (e) further comprises the step of subjecting said isolated double-labelled polypeptide fraction to collision-induced dissociation (CID) and determining the relative amount of different isobarically labelled polypeptides therein.

16. The method of claim 15, wherein the relative amount of different isobarically labelled polypeptide is determined by determining the relative amount of reporter groups released from the isobaric label after CID.

17. The method of claim 16, further comprising the step of determining the sequence of said polypeptide.

18. A method for double-labelling a polypeptide sample with at an isobaric and an isotopic labelling reagent comprising the steps of:
- reacting in a first labelling step a first labelling reagent with a first functional group on the polypeptides in said sample,
- reacting in a second labelling step the second labelling reagent with a second functional group on the polypeptides in said sample.
wherein one of the first and the second labelling reagent is an isobaric reagent and the other is an isotopic reagent,

19. Use of the method of according to any of claims 1 to 18 for identifying proteins which are differentially expressed between a protein sample of a control individual and one or more experimental protein samples.

20. A polypeptide or peptide comprising one of a set of isotopic labels on a first functional group of said polypeptide and one of a set of isobaric labels on a second functional group of said polypeptide.

21. The polypeptide or peptide of claim 20, wherein one of the isotopic or isobaric label is on the side chain of an amino acid in said polypeptide and wherein the other of the isotopic or isobaric label is on the aminoterminus or the carboxyterminus of said polypeptide.

22. The polypeptide or peptide according to claim 20, wherein the first functional group is a thiol and wherein the second functional group is a primary amine.

23. The polypeptide or peptide according to claim 22 wherein said thiol is the thiol of a cysteine in said polypeptide and said primary amine is the primary amine group of the aminoterminus of said polypeptide.

24. The peptide according to claim 20, which is tryptic peptide of a protein.

25. The peptide or polypeptide according to claim 20, wherein one the isotopic or isobaric label is on the aminoterminus of said polypeptide and wherein the other of the isotopic or isobaric label is on the carboxyterminus of said polypeptide.

26. The polypeptide according to claim 20 wherein the general structure of the isotopic label on the polypeptide corresponds to: 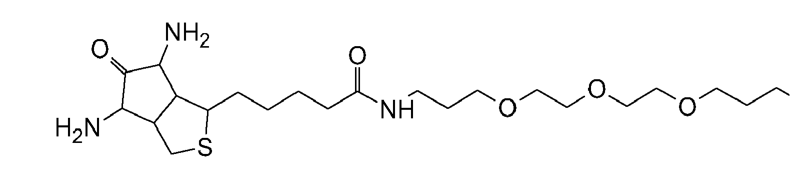 Or:

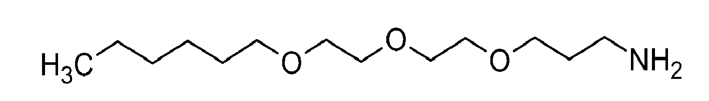

27. The polypeptide according to claim 21 wherein the isobaric label on the polypeptide has a general structure:

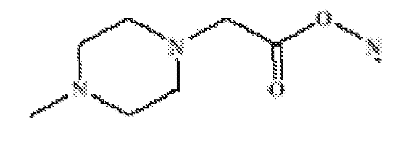

28. A mixture of polypeptides, said mixture comprising a one or plurality of polypeptides according to claim 20, wherein polypeptides with an identical amino acid sequence carry different combinations of different isotopic labels with a same chemical formula and different isobaric labels with a same chemical formula.

29. The mixture according to claim 28, wherein the number of different isobaric labels is at least 2 and wherein the number of different isobaric labels is at least 2.

30. The mixture according to claim 28 wherein the number of different isobaric labels is 4 and the number of different isotopic labels is 2.

31. The mixture according to claim 28 wherein the number of different isobaric labels is 8 and the number of different isotopic labels is 2.

32. A kit of reagents comprising at least one isotopic labelling reagent and at least one isobaric labelling reagent, wherein said reagents have protein reactive groups.

33. The kit of reagents wherein one said labelling reagents comprises an affinity tag.

34. A method of determining relative amounts of individual polypeptides or peptides from different samples with the same amino acid sequence in a pooled mixture of these, wherein each individual polypeptide or peptide from a different sample is double labelled with a different combination of one of a set of isotopic labels and one of a set of isobaric labels, comprising the steps of:
a) optionally isolating the polypeptides or peptides by liquid chromatography, so as to obtain an isolated fraction comprising said individual polypeptides or peptides having the same amino acid sequence,
b) separating the peptides or polypeptides in the pooled mixture or the isolated fraction of polypeptides obtained in step (a) into further fractions of polypeptides or peptides with different mass, by a first MS and determining the relative amount of each polypeptide or peptide fraction with a different mass.
c) subjecting the peptide fractions obtained in step (b) to a second MS, under conditions wherein the isobaric labels of the individual polypeptides are fragmented and determining the amounts of each of the fragmented isobaric labels of the individual polypeptides or peptides within the polypeptide or peptide fractions.
d) determining from the amount of each of the isobaric labels determined in step (c), the relative amount of the individual polypeptide or peptide within a polypeptide or peptide fraction obtained in step (b),
e) calculating from the relative amounts of each fraction with a different mass determined in step (b) and from the relative amounts of the individual peptides determined in step (d), the relative amount of each individual polypeptide or peptide having the same amino acid sequence present in said pooled mixture.

35. The method according to claim 34, wherein each individual polypeptide or peptide from a different sample is double labelled with a different combination of ¹⁸O or ¹⁶O at the carboxyterminus and one of a set of isobaric labels.

36. A device (100) for multiplex analysis of protein samples using double labelling comprising at least two sources of samples (101), two labelling units (103 and 103') and corresponding label sources (104 and 107) each comprising a set of reagents suitable for isotopic or isobaric labelling, a separation unit (108), a mass spectrometer unit (109) and a data analysis unit (110).

37. The device according to claim 36 further comprising one or more elements selected from the group consisting of a sample preparation unit (102), a protein cleavage unit (105) and an affinity purification unit (106).

38. A method for simultaneously analysing the presence of one or more polypeptides comprising a functional group in different samples, which comprises the steps of:
a) a first labelling step wherein at least one sample is isotopically labelled by protease mediated ¹⁸O incorporation into the C-terminus of the generated therefrom,
b) a second labelling step wherein a set of isobaric labelling reagents, is reacted with a functional group on the polypeptides in the samples, or peptides generated therefrom wherein the combination of labelling steps is performed such that each of the samples is labelled with a different combination of O isotope and isobaric.
c) pooling the different samples to obtain a polypeptide sample mix selectively isolating the double-labelled polypeptides from the polypeptide sample mix
d) analysing the relative occurrence of the isolated double-labelled polypeptides by Mass spectroscopy.

39. The method according to claim 38, wherein the set of labelling isobaric reagents comprises an affinity tag.

40. The method according to claim 38, wherein the protease is trypsin.

41. The method according to claim 39, wherein the peptides are isolated using the affinity tag which is introduced onto the peptides by way of the isobaric labelling reagents.

42. The method according to claim 38, wherein the functional group is a thiol.

43. The method according to claim 38, wherein the set of isobaric labelling reagents all comprise a molecule comprising a methyl piperazine derived moiety, a carbonyl moiety and a protein reactive moiety with the formula 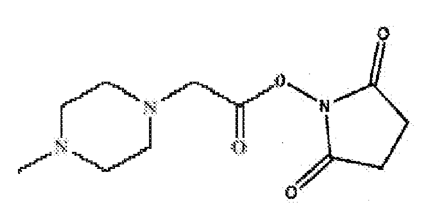 wherein one or more of the atoms in the methyl piperazine derived moiety or in the carbonyl moiety is an isotope selected from the group consisting of ¹⁸O, ²H, ¹⁵N and ¹³C.

44. The method of claim 38, wherein step (d) comprises the step of isolating a double-labelled peptide fraction from said pooled polypeptides by electrophoresis or chromatography.

45. The method of claim 38, wherein step (e) comprises the step of determining with MS the relative amount of different isotopes in said isolated double-labelled peptide fraction.

46. The method of claim 45, wherein step (e) further comprises the step of subjecting said isolated double-labelled polypeptide fraction to collision-induced dissociation (CID) and determining the relative amount of different isobarically labelled polypeptides therein.

47. The method of claim 46, wherein the relative amount of different isobarically labelled polypeptide is determined by determining the relative amount of reporter groups released from the isobaric label after CID.

48. The method of claim 47, further comprising the step of determining the sequence of said polypeptide.

49. A method for double-labelling a polypeptide sample with an isobaric labelling reagent and ¹⁸O comprising the steps of:
a) treating the polypeptide sample with a protease and H₂¹⁸O, thereby incorporating in a first labelling step ¹⁸O in the C-terminus of proteolytic peptides of the polypeptides in said sample,
b) reacting in a second labelling step an isobaric labelling reagent with a functional group on the polypeptides in said sample.

50. Use of the method according to any of claims 38 to 49 for identifying proteins which are differentially expressed between a protein sample of a control individual and one or more experimental protein samples.

51. A peptide comprising one or two ¹⁸O atoms at the C-terminus and one of a set of isobaric labels on a functional group of said polypeptide.

52. The peptide according to claim 51, wherein the functional group is a thiol.

53. The peptide according to claim 51, which is tryptic peptide of a protein.

54. The polypeptide according to claim 51, wherein the one of a set of isobaric labels on the polypeptide has a general structure:

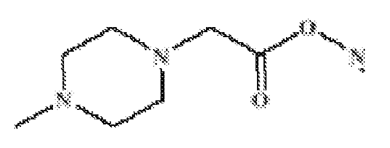

55. A mixture of polypeptides, said mixture comprising one or plurality of polypeptides according to claim 51 wherein polypeptides with an identical amino acid sequence carry different combinations of ¹⁸O isotopic labelled C-termini and different isobaric labels with a same chemical formula.

56. The mixture according to claim 55, wherein the number of different isobaric labels is at least 2.

57. A kit of reagents comprising H₂¹⁸O and a set of at least two isobaric labelling reagents, wherein said reagents comprise a protein reactive group.

58. The kit of reagents according to claim 57 wherein said isobaric labelling reagents comprise an affinity tag.
